# EUROPEAN PATENT SPECIFICATION

(11) **EP 2 340 003 B1**
(45) Date of publication and mention of the grant of the patent: **24.07.2013**
(21) Application number: 10748177.2
(22) Date of filing: 19.08.2010
(51) Int. Cl.: A61F 13/15, B05C 1/16, B41F 5/24

(54) **ABSORBENT ARTICLES HAVING BOTH DISTINCT AND IDENTICAL GRAPHICS AND APPARATUS AND METHOD FOR PRINTING SUCH ABSORBENT ARTICLES**
ABSORBIERENDE ARTIKEL, DIE SOWOHL UNTERSCHIEDLICHE UND IDENTISCHE GRAFIKEN HABEN UND VORRICHTUNG UND VERFAHREN ZUM BEDRUCKEN SOLCHER ABSORBIERENDEN ARTIKEL
ARTICLES ABSORBANTS AYANT DEUX TYPES DE GRAPHIQUES DISCTINCTS ET IDENTIQUES, APPAREIL ET PROCEDE POUR IMPRIMER DE TELS ARTICLES

(30) Priority: 21.08.2009 US 235845 P
(43) Date of publication of application: 06.07.2011
(73) Proprietor: The Procter & Gamble Company, Cincinnati, OH 45202 (US)
(72) Inventor: WARNER, Alrick, Vincent, Loveland Ohio 45140 (US)
(74) Representative: Borbach, Markus
(86) International application number: PCT/US2010/045987
(87) International publication number: WO 2011/022537

(56) References cited:
- WO-A1-2005/102237
- WO-A1-2006/007031
- WO-A2-2004/064872
- US-A1- 2005 015 066
- US-A1- 2005 067 083

## Description

### FIELD OF THE INVENTION

The present disclosure relates to printing apparatuses and methods for printing absorbent articles, and more particularly, apparatuses and methods for the flexographic printing of a series of different graphics on a plurality of absorbent articles as well as products including such absorbent articles.

### BACKGROUND OF THE INVENTION

Along an assembly line, diapers and various types of other disposable absorbent articles may be assembled by adding components to and otherwise modifying an advancing, continuous web of material. Webs of material and component parts used to manufacture diapers may include: backsheets, topsheets, absorbent cores, front and/or back ears, fastener components, and various types of elastic webs and components such as leg elastics, barrier leg cuff elastics, and waist elastics. In some processes, graphics are printed on individual components and/or continuous webs of material used to assemble the absorbent articles.

Some consumers may prefer purchasing absorbent articles, such as diapers, having a number of different graphic designs printed thereon and provided in a single package. Various methods and apparatuses can be used to print different graphics on an advancing web of material used in the manufacture of absorbent articles. However, such methods and apparatuses provide for limited numbers of different printed graphics, graphics with relatively low quality print, and/or require relatively low print and/or manufacture speeds. In addition, such methods and apparatuses may also require relatively expensive processes and equipment and may not be very flexible in allowing a user to change the type of graphics to be printed. Further, such apparatuses may be rather large and take up excessive amounts of space.

### SUMMARY OF THE INVENTION

Aspects of the present disclosure involve apparatuses and methods for manufacturing absorbent articles, and more particularly, for printing graphics on substrates during the manufacture of components of absorbent articles. Such graphics include a series of different or distinct graphics in combination with a series of identical graphics. As discussed below, examples of such printed substrates can be used in the manufacture of printed diaper components, such as for example, backsheets, topsheets, landing zones, fasteners, ears, absorbent cores, and acquisition layers.

In one form, an apparatus for printing disposable absorbent articles includes: a central impression cylinder defining an outer circumferential surface; a constant graphic printing station positioned adjacent the outer circumferential surface of the central impression cylinder; a first variable graphic printing station positioned adjacent the outer circumferential surface of the central impression cylinder; a second variable graphic printing station positioned adjacent the outer circumferential surface of the central impression cylinder; and a third variable graphic printing station positioned adjacent the outer circumferential surface of the central impression cylinder. The constant graphic printing station includes: a print cylinder defining on outer circumferential surface defining a first circumferential length; a constant graphic printing pattern disposed on the outer circumferential surface of the print cylinder; an ink supply; and an anilox roller operably connected with the ink supply and the print cylinder wherein the anilox roller is adapted to deposit ink from the ink supply onto the constant graphic printing pattern. Each variable graphic printing station includes: a print cylinder defining on outer circumferential surface defining a second circumferential length; a plurality of n variable printing patterns disposed on the outer circumferential surface of the print cylinder, wherein n is 2 or greater and wherein the n variable printing patterns are different from each other; an ink supply; and an anilox roller operably connected with the ink supply and the print cylinder wherein the anilox roller is adapted to deposit ink from the ink supply onto the plurality of n variable printing patterns. The constant graphic printing pattern is different from the n variable printing patterns; and the second circumferential length is at least two times the first circumferential length.

In another form, a method for producing disposable absorbent articles includes the steps of: feeding a substrate onto a rotating central impression cylinder; moving the substrate past a constant graphic printing station arranged adjacent an outer surface of the central impression cylinder, wherein the constant graphic printing station includes print cylinder with a plurality of identical printing patterns operably disposed thereon; rotating the print cylinder of the constant graphic printing station to print a series of identical graphics on the substrate; moving the substrate past a plurality of variable graphic printing stations arranged around an outer surface of the central impression cylinder, wherein each printing station includes a print cylinder with n variable printing patterns operably disposed thereon, wherein n is 2 or greater and wherein the n variable printing patterns are different from each other and are different from the identical printing patterns; rotating the print cylinders of the variable graphic printing stations to print a series of n graphics adjacent the identical graphics; converting the substrate into printed components of disposable absorbent articles; and placing the disposable absorbent articles into a package.

In yet another form, a disposable absorbent product includes: a package; at least n disposable absorbent articles contained in the package, wherein n is 4 or greater and wherein each of the disposable absorbent articles includes: a topsheet; a backsheet; and an absorbent core disposed between the topsheet and the backsheet, the topsheet and the backsheet; and a first graphic printed directly on at least one of the backsheet, the absorbent core, and the topsheet; and a second graphic printed adjacent the first graphic. The first graphics of each of the n disposable absorbent articles are different from each other; the second graphics of each of the n disposable absorbent articles are identical to each other; and each first graphic comprises: a first ink color printed in first rows of first dots at a first screen angle, and a second ink color printed in second rows of second dots at a second screen angle, and wherein the second dots are printed such that portions of the second dots overlap portions of the first dots; and wherein each second graphic is a spot color graphic.

In still another form, a series of disposable absorbent products includes: at least m packages, wherein m is 2 or greater, wherein each package includes a first package graphic and a second package graphic printed thereon, and wherein the first package graphics printed on each of the m packages are different from each other and wherein the second package graphics printed on each of the m packages are identical to each other; at least n disposable absorbent articles contained in each package, wherein n is 2 or greater and wherein each of the disposable absorbent articles includes: a topsheet; a backsheet; and an absorbent core disposed between the topsheet and the backsheet, the topsheet and the backsheet; and a first article graphic printed directly on at least one of the backsheet, the absorbent core, and the topsheet; a second article graphic printed directly on at least one of the backsheet, the absorbent core, and the topsheet; and wherein the first article graphics of each of the n disposable absorbent articles are different from each other; wherein the second article graphics of each of the n disposable absorbent articles are identical to each other; and wherein each first package graphic and each first article graphic comprises: a first ink color printed in first rows of first dots at a first screen angle, and a second ink color printed in second rows of second dots at a second screen angle, and wherein the second dots are printed such that portions of the second dots overlap portions of the first dots.

### BRIEF DESCRIPTION OF THE DRAWINGS

Fig. 1 is a schematic view of printing apparatus according to the present disclosure.
Fig. 2A is a detailed schematic view of a constant graphic printing station.
Fig. 2B is a partial detailed side view of a print cylinder and associated printing plates of a constant graphic printing station from Fig. 2A.
Fig. 2C is a top side view of a printing plate from Fig. 2B.
Fig. 3A is a detailed schematic view of a variable graphic printing station.
Fig. 3B is a partial detailed side view of a print cylinder and associated printing plates of a variable graphic printing station from Fig. 3A.
Fig. 3C is a top side view of a printing plate from Fig. 3B.
Fig. 4A is a top view of a substrate with a sample series of identical graphics printed thereon.
Fig. 4B is a top view of a substrate with a sample series of variable graphics printed thereon.
Fig. 4C is a top view of a substrate with a sample series of variable graphics printed thereon.
Fig. 4D is a partial view of a print cylinder with a plurality of printing plates arranged in the CD and MD directions.
Fig. 5 shows an example of ink dots utilized with halftone printing.
Fig. 6A is a perspective view an absorbent product.
Fig. 6B shows a series of diapers from the absorbent product of Fig. 6A.
Fig. 7 is a perspective view an absorbent article.
Fig. 8 is a partially cut away plan view of the absorbent article shown in Fig. 7.
Fig. 9 shows a series of packages for consumer products.

### DETAILED DESCRIPTION OF THE INVENTION

The following term explanations may be useful in understanding the present disclosure:

"Absorbent article" is used herein to refer to consumer products whose primary function is to absorb and retain soils and wastes. Non-limiting examples of incontinent absorbent articles include diapers such as PAMPERS diapers, training and pull-on pants such as PAMPERS FEEL 'N LEARN and EASY UPS, adult incontinence briefs and undergarments such as ATTENDS adult incontinence garments, feminine hygiene garments such as panty liners, absorbent inserts, and the like such as ALWAYS and TAMPAX, all sold by The Procter & Gamble Company.

"Diaper" is used herein to refer to an absorbent article generally worn by infants and incontinent persons about the lower torso.

The term "disposable" is used herein to describe absorbent articles which generally are not intended to be laundered or otherwise restored or reused as an absorbent article (e.g., they are intended to be discarded after a single use and may also be configured to be recycled, composted or otherwise disposed of in an environmentally compatible manner).

The term "disposed" is used herein to mean that an element(s) is formed (joined and positioned) in a particular place or position as a macro-unitary structure with other elements or as a separate element joined to another element.

As used herein, the term "joined" encompasses configurations whereby an element is directly secured to another element by affixing the element directly to the other element, and configurations whereby an element is indirectly secured to another element by affixing the element to intermediate member(s) which in turn are affixed to the other element.

The term "substrate" is used herein to describe a material which is primarily two-dimensional (i.e. in an XY plane) and whose thickness (in a Z direction) is relatively small (i.e. 1/10 or less) in comparison to its length (in an X direction) and width (in a Y direction). Non-limiting examples of substrates include a layer or layers or fibrous materials, films and foils such as plastic films or metallic foils that may be used alone or laminated to one or more web, layer, film and/or foil. As such, a web is a substrate.

The term "nonwoven" refers herein to a material made from continuous (long) filaments (fibers) and/or discontinuous (short) filaments (fibers) by processes such as spunbonding, meltblowing, and the like. Nonwovens do not have a woven or knitted filament pattern.

The term "machine direction" (MD) is used herein to refer to the direction of material flow through a process.

The term "cross direction" (CD) is used herein to refer to a direction that is generally perpendicular to the machine direction.

The terms "elastic" and "elastomeric" as used herein refer to any material that upon application of a biasing force, can stretch to an elongated length of at least about 110% of its relaxed, original length (i.e. can stretch to 10 % more than its original length), without rupture or breakage, and upon release of the applied force, recovers at least about 40% of its elongation. For example, a material that has an initial length of 100 mm can extend at least to 110 mm, and upon removal of the force would retract to a length of 106 mm (40% recovery). The term "inelastic" refers herein to any material that does not fall within the definition of "elastic" above.

The term "extensible" as used herein refers to any material that upon application of a biasing force can stretch to an elongated length of at least about 110% of its relaxed, original length (i.e. can stretch to 10 %), without rupture or breakage, and upon release of the applied force, shows little recovery, less than about 40% of its elongation.

The terms "activating", "activation" or "mechanical activation" refer to the process of making a substrate, or an elastomeric laminate more extensible than it was prior to the process.

"Live stretch" includes stretching elastic and bonding the stretched elastic to a substrate. After bonding, the stretched elastic is released causing it to contract, resulting in a "corrugated" substrate. The corrugated substrate can stretch as the corrugated portion is pulled to about the point that the substrate reaches at least one original flat dimension. However, if the substrate is also elastic, then the substrate can stretch beyond the relaxed length of the substrate prior to bonding with the elastic. The elastic is stretched at least 25% of its relaxed length when it is bonded to the substrate.

The term "body facing surface" refers to surfaces of absorbent articles and/or components thereof which face a wearer's body when the absorbent articles are worn, and the term "garment facing surface" refers to surfaces of absorbent articles and/or components thereof that face away from a wearer's body when the absorbent articles are worn. Absorbent articles and components thereof, including the topsheet, backsheet, absorbent core, and any individual materials of their components, have a body facing surface and a garment facing surface.

The term "graphic" refers to images or designs that are constituted by a figure (e.g., a line(s)), a symbol or character, a color difference or transition of at least two colors, or the like. A graphic may include an aesthetic image or design that can provide certain benefit(s) when an absorbent article is viewed.

Aspects of the present disclosure involve apparatuses and methods for manufacturing absorbent articles, and more particularly, for printing graphics on substrates during the manufacture of components of absorbent articles. Such graphics include a series of different or distinct graphics in combination with a series of identical graphics. As discussed below, examples of such printed substrates can be used in the manufacture of printed diaper components, such as for example, backsheets, topsheets, landing zones, fasteners, ears, absorbent cores, and acquisition layers. Although the description below is mainly related to diaper components, it is to be appreciated that the apparatuses and methods discussed herein are also applicable to other types of absorbent articles. Particular embodiments of the apparatuses and methods disclosed herein utilize flexographic printing to provide for a sequential manufacture of a series of n absorbent articles having different graphics printed thereon, wherein n can be a number of 2 or greater. In some embodiments, n can be a number of 6 or greater. In addition, one or more, or a portion, of the series of n absorbent articles may be placed in a package to produce an absorbent product.

In one implementation, during the manufacture of absorbent article components, a substrate traveling in a machine direction (MD) is fed onto a rotating central impression cylinder or drum of a flexographic printing apparatus. A plurality of printing stations is located around a portion of the outer circumference of the central impression cylinder. In particular, the plurality of printing stations includes variable graphic printing stations and constant graphic printing stations. While disposed on the rotating central impression cylinder, the substrate moves past the printing stations. The constant graphic printing stations print a series of identical graphics IG on the substrate. And the variable graphic printing stations, in turn, print a repeating series of n graphics (G1-Gn) on the substrate adjacent the identical graphics, wherein each of the n graphics is different from each other, wherein n can be a number of 2 or greater and in some embodiments, n can be a number of 6 or greater. During the manufacture of absorbent articles, the printed substrate may be used to manufacture individual printed components and modified or otherwise combined with other advancing substrates or webs and/or individual component parts. Once the desired component parts are assembled, the advancing web(s) are subjected to a final knife cut to separate the web(s) into discrete absorbent articles, such as diapers. Thus, the discrete absorbent articles are manufactured such that a repeating series of n adjacent articles each have different graphics printed thereon. Thus, an absorbent product may be manufactured by folding, stacking, and placing one or more, or a portion of, the series of n absorbent articles in a package.

As discussed in more detail below, each printing station of the printing apparatus includes a print cylinder. A plurality of flexible printing plates may be disposed on an outer surface of the print cylinder. On the constant graphic print stations, each flexible printing plate includes an identical graphic pattern corresponding to an identical graphic to be printed on the substrate. As the central impression cylinder rotates, the substrate is advanced into a nip between the central impression cylinder and each printing station. At the same time, rotation of the central impression cylinder causes the print cylinders to rotate and advances associated printing plates into contact with the substrate.

As the print cylinder on the constant print station rotates, a first printing plate moves into contact with the substrate to print a first graphic onto the substrate. As the central impression cylinder continues to rotate, the substrate continues to move past the printing station, and the print cylinder rotates to advance a second printing plate into contact with the substrate to print a second associated graphic onto the substrate, which is identical to the first graphic. As the substrate advances, the print cylinder continues to rotate and continues to print a series of identical graphics along the length of the substrate.

As the print cylinder on the variable print station rotates, a first printing plate moves into contact with the substrate to print a first associated graphic onto the substrate. As the central impression cylinder continues to rotate, the substrate continues to move past the printing station, and the print cylinder rotates to advance a second printing plate into contact with the substrate to print a second associated graphic onto the substrate. The central impression cylinder continues to rotate and the print cylinder continuously rotates such that all n printing plates disposed on the print cylinder print associated graphics onto the substrate. As a result, a series of n graphics (G₁-Gₙ) is printed on the substrate, wherein each of the n graphics may be different from each other. Once all n graphics are printed on the substrate, the print cylinder rotates to advance to the first printing plate into contact with the substrate again and continues to repeatedly print the series of graphics.

As discussed below, the variable and constant graphic printing stations can be configured in various ways to print different colored graphics. For example, in one embodiment, the printing stations may be configured to print graphics on a substrate through a process of halftone process printing. In other embodiments, the constant graphic print stations may be configured to print single colors, such as in spot color printing, whereas the variable graphic printing stations are configured to print different colors, such as in a CMYK printing arrangement. Spot color may be a specific color in a design or graphic to be printed with a specific matching ink and a single printing plate rather than through process CMYK printing. For example, a teal spot color may have a Pantone Matching System (PMS) number of 3272 and/or a Hunter Color CIELAB color values of L*=63.4, a*=-56.25, and b*=-7.74.

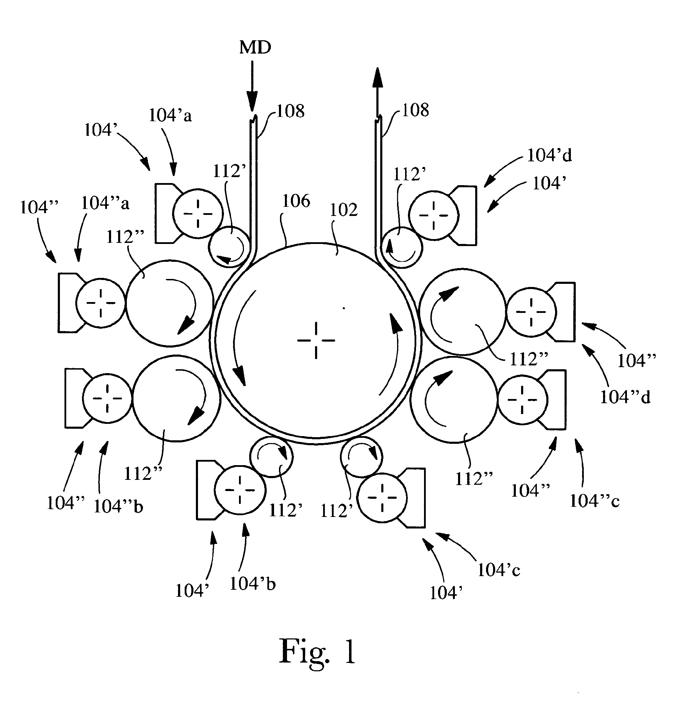

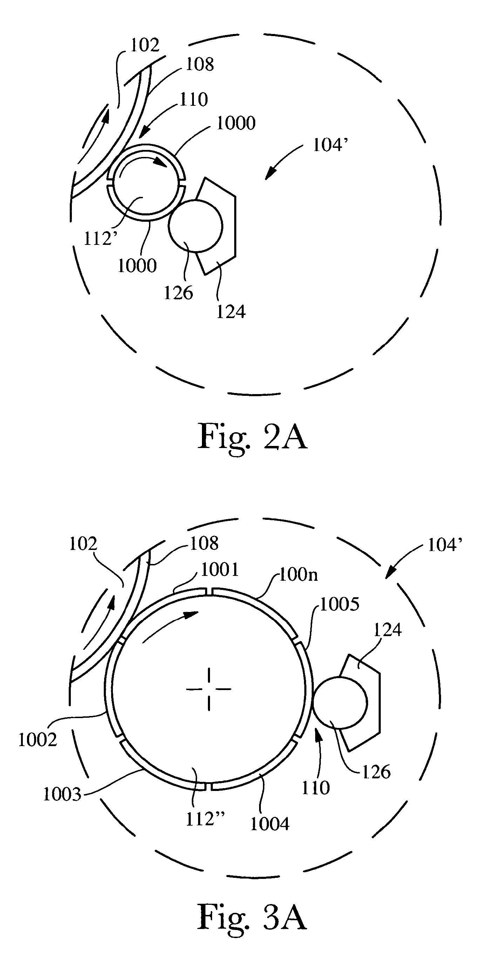

Fig. 1 shows an embodiment of a printing apparatus 100 conforming to aspects of the present disclosure. As shown in Fig. 1, the printing apparatus 100 includes a central impression cylinder (CIC) or CI drum 102 and a plurality of printing stations 104 disposed along an outer surface 106 of the central impression cylinder 102. The printing stations 104 include constant graphic printing stations 104' and variable graphic printing stations 104". Figs. 2A-3C show detailed views of embodiments of the printing stations 104', 104" and embodiments of various components associated therewith. Although the printing apparatus 100 shown in Fig. 1 includes four constant graphic printing stations 104' and four variable printing stations 104", it is to be appreciated that other embodiments may include more or less than four of each type of printing stations.

Referring back to Fig. 1, in operation, the central impression cylinder 102 rotates in the direction shown and a substrate 108 is fed onto the rotating central impression cylinder 102, which moves past each printing station 104', 104" and exits the printing apparatus. As the substrate moves 108 past the constant graphic printing stations 104', the constant graphic printing stations 104' print a series of identical graphics IG onto the substrate 108. And the variable printing stations 104" print a series of graphics (G₁-Gₙ) onto the substrate 108. As discussed in more detail below, some components of the printing stations are located relatively close to the outer surface 106 of the central impression cylinder 102 so as to create a nip 110 between each printing station 104 and the central impression cylinder 102. The nips 110 help maintain the substrate 108 in a constant or fixed position relative to the outer surface 106 of central impression cylinder 102, which in turn, helps provide print registration control.

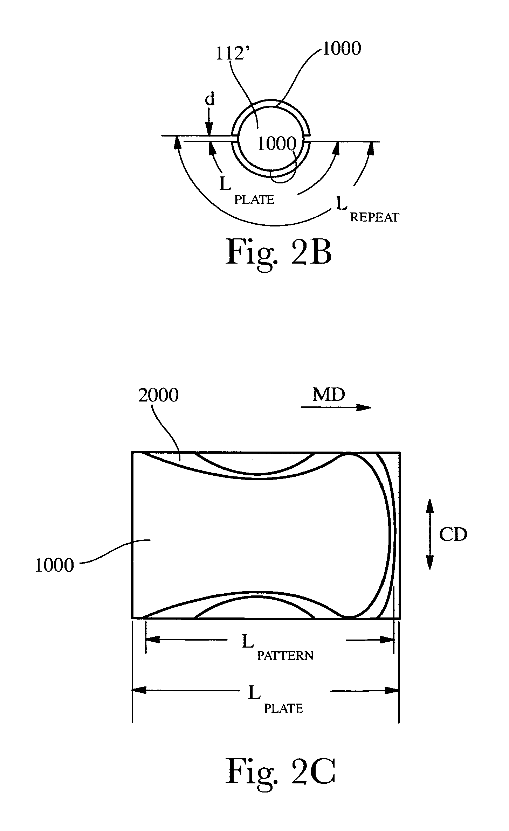

As previously mentioned, the constant graphic printing stations can be configured to print a repeating series of identical graphics on the substrate. As shown in Figs. 2A-2C, each constant graphic printing station 104' includes print cylinder 112' with a plurality of flexible printing plates 1000 disposed thereon. As shown in Fig. 2B, the print cylinder 112' has an outer circumferential surface 120 with the printing plates 1000 disposed thereon. As discussed in more detail below, the printing plates 1000 may include constant graphic printing patterns 2000 that are identical to each other. During operation, the central impression cylinder 106 rotates and causes the print cylinder 112' to rotate and advance the printing plates 1000 on the print cylinder to move into contact with the substrate 108 disposed on the rotating central drum 102. As discussed below, as the printing plates move into contact with the substrate, ink on the printing patterns is transferred to the substrate to print a series of identical graphics thereon.

In contrast to the constant graphic printing stations, the variable graphic printing stations can be configured to print a repeating series of different graphics on the substrate. As shown in Figs. 3A-3C, each printing station 104" includes a print cylinder 112" having an outer circumferential surface 120 with a plurality of flexible printing plates (1001-100n) disposed thereon. As discussed in more detail below, the printing plates (1001-100n) may include printing patterns (2001-200n) that are different from each other. During operation, the central impression cylinder 106 rotates and causes the print cylinder 112" to rotate and advance the printing plates into contact with the substrate 108 disposed on the rotating central drum 102. As discussed below, as the printing plates move into contact with the substrate, ink on the printing patterns is transferred to the substrate to print a series of different graphics thereon.

It is to be appreciated that the print cylinders of the printing stations may include various numbers of printing plates. For example, in one embodiment, the variable graphic printing stations may include four printing plates adapted to print a repeating series of four different graphics on the substrate. In another embodiment, the variable graphic printing stations may include six printing plates adapted to print a repeating series of five different graphics on the substrate. Thus, the number of printing plates may correspond with the number of different graphics intended to be printed on the substrate. As such, a larger number of different graphics may require a correspondingly larger number of printing plates, and in turn, may require print cylinder having a correspondingly larger diameter. In contrast to the variable graphic printing stations, the printing plates of the constant graphic printing stations repeatedly print the same graphics. Thus, the number of printing plates can be independent of the number of different graphics to be printed. For example, in some embodiments, the constant graphic printing stations may include 1, 2, or 3 printing plates while the variable graphic printing plates include 2, 4, 5, or 6 printing plates. As such, the diameters of the print cylinders of the constant graphic printing stations can be smaller than the diameters of the print cylinders of the variable graphic printing stations.

In addition, the printing apparatus may be configured with one or more constant graphic printing stations having print cylinders that are relatively small, and the one or more variable graphic printing stations that having print cylinders that are relatively large. The printing plates on the variable graphic printing stations may be configured only with patterns that print variable graphics, whereas printing plates on the constant graphic printing plates may be configured only with printing patterns that print identical graphics. Because the printing plates on the variable graphic printing stations need not print both identical graphics and variable graphics, the printing plates on the variable graphic printing stations may have relatively less complex patterns thereon. And in a scenario wherein it is desirable to change the variable graphics while keeping the identical graphics the same (such as during seasonal product offerings), the printing plates on the variable graphic printing station can be changed without having to replace to the printing plates on the constant printing stations.

As shown in Figs. 2A and 3A, the constant graphic printing stations 104' and the variable graphic printing stations 104" may each include an ink supply 124 and an anilox roller 126, which is operably connected with the ink supply 124 and the print cylinder 112. During operation of the printing apparatus 100, the anilox roller 126 rotates and deposits ink from the ink supply 124 onto the printing plates 1000, (1001-100n) on the print cylinder 112', 112". More particularly, the anilox roller 126 transfers ink onto printing patterns 2000, (2001-200n) on the printing plates as the printing plates move past the anilox roller. The printing stations 104', 104" may also include a device to remove excess ink from the anilox roller. For example, in some embodiments, the printing stations include a doctor blade configured to scrape excess ink from the anilox roller before transferring ink to the printing plates. As the print cylinder 112', 112" rotates, the printing plates 1000, (1001-100n) move into contact with the substrate 108 on the central drum 102, and in turn, transfer ink from the printing patterns 2000, (2001-200n) on the printing plates to the substrate. Although not shown in Figs. 2A and 3A, it is to be appreciated that the printing stations 104', 104" may also include ink driers. Driers located between printing stations may serve to partially dry the ink printed by a preceding print station, which may fix the ink from each preceding print station to the substrate and to help minimize ink smearing.

It is to be appreciated that various types and configurations of printing plates may be used. For example, in some embodiments, the printing plates may be constructed from flexible photopolymer or rubber. The printing patterns may be formed on the printing plates in various ways. For example, in some embodiments, the printing patterns are engraved into the printing plates. It should also be appreciated that the printing plates can be secured to the outer surface of the print cylinder in various ways, such as with, for example, fasteners, adhesives, and tape. In some embodiments, the printing stations do not include printing plates, and instead, include print cylinders having printing patterns formed directly therein. As previously mentioned, graphics are printed on the substrate when ink is transferred from the printing patterns on the printing plates to the substrate. As such, the CD width and MD length of the printed graphics can also be varied by varying the size of the printing patterns on the printing plates. For example, some embodiments can be configured to print graphics having a CD width of 2.5 m or greater. As mentioned above, some embodiments of printing stations can be configured with various numbers of printing plates, and as such, may accommodate different diameters of print cylinders.

As mentioned above, the printing apparatuses 100 according to the present disclosure can be configured to print a repeating series of n different graphics (G1-Gn) in combination with identical graphics IG on a substrate. In operation, the central impression cylinder 102 rotates in the direction shown for example in Fig. 1, and the substrate 108 is fed onto the rotating central impression cylinder 102. In turn, the substrate 108 moves past each printing station 104 as the central impression cylinder 102 rotates. As the substrate moves past the constant graphic printing stations 104', a series of identical graphics are printing on the substrate. And as the substrate moves past the variable graphic printing stations 104", the printing stations 104" print a series of different graphics (G1-Gn) onto the substrate 102 adjacent the identical graphics IG.

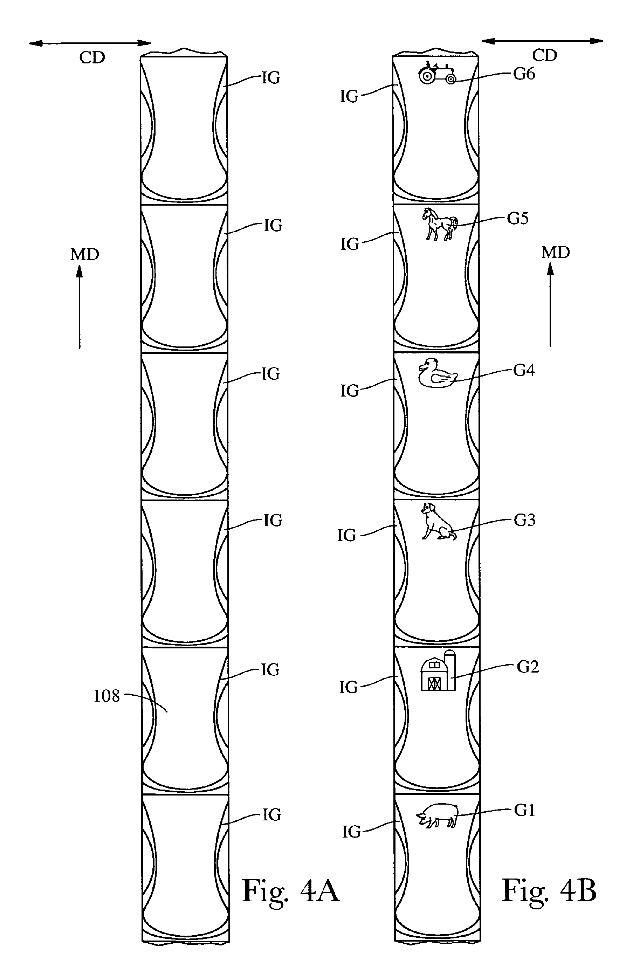

With regard to the constant graphic printing stations 104', the rotation of the central impression cylinder 102 causes the print cylinder 112' on each constant graphic printing station 104' to rotate. As the print cylinder rotates, each printing plate 1000 is sequentially moved into contact with the anilox roller 126, which transfers ink onto the printing patterns 2000. At the same time, each printing plate 1000 is sequentially moved into contact with the substrate 108 on the rotating central impression cylinder 102. Identical graphics IG are printed on the substrate 102 as ink from the printing patterns 2000 on the printing plates 1000 is transferred to the substrate 108. Thus, a repeating series of identical graphics are printed on the substrate along the MD direction once each printing plate has been moved into contact with substrate. Fig. 4A shows an example of a substrate 108 printed with a repeating series of 6 identical graphics IG in the MD direction. The identical graphics shown in Fig. 4A are in the form of a curved stripe pattern having a varying width. It is to be appreciated that the identical graphics may have various forms and may perform various functions. For example, in some instances, the identical graphics IG may help communicate a product brand identity or equity to a consumer through its shape, color, and/or size. In another example, the identical graphics IG may be used as registration marks. In yet another example, the identical graphics IG may be provided in the form of and used as serviceable indicia, such as disclosed in U.S. Patent Publication Nos. US2007/0142798A1 and US2008/0004585A1, which are both incorporated by reference herein.

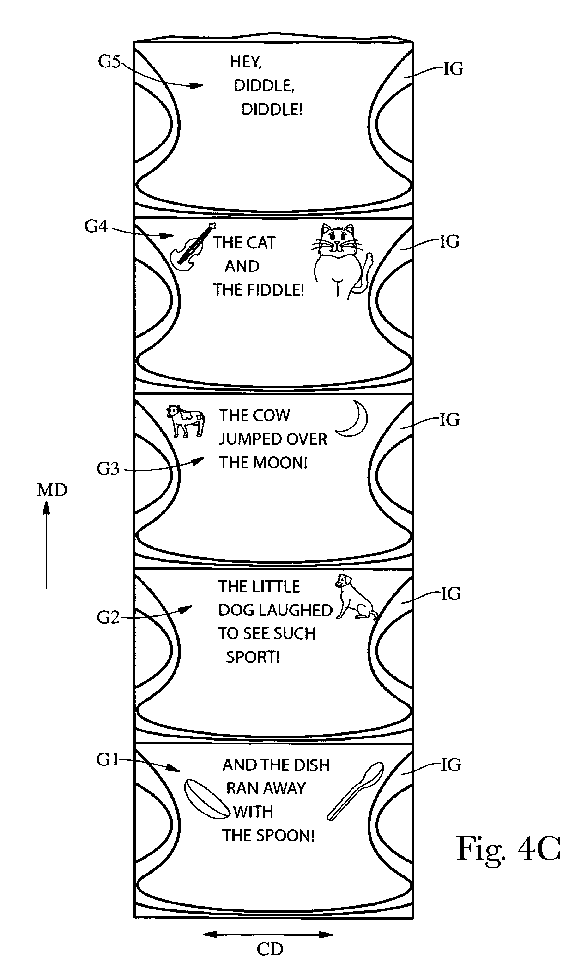

In conjunction with the identical graphics IG printed by the constant graphic print stations 104', the rotation of the central impression cylinder 102 causes the print cylinder 112" on each variable graphic printing station 104" to rotate. As the print cylinder rotates, each printing plate (1001-100n) is sequentially moved into contact with the anilox roller 126, which transfers ink onto the printing patterns (2001-200n). At the same time, each printing plate (1001-100n) is sequentially moved into contact with the substrate 108 on the rotating central impression cylinder 102. Graphics (G1-Gn) are printed on the substrate 102 as ink from the printing patterns (2001-200n) on the printing plates (1001-100n) is transferred to the substrate 108. Thus, a repeating series of different graphics are printed on the substrate along the MD direction once each printing plate has been moved into contact with substrate. For example, printing stations having n printing plates (1001-100n) may print a repeating series of n graphics (G1-Gn) on the substrate. Fig. 4B shows an example of a substrate 108 printed with a repeating series of 6 different graphics (G1-G6) in the MD direction wherein the repeating series of 6 different graphics (G1-G6) are printed adjacent the identical graphics IG from the constant graphic printing stations 104'. Fig. 4C shows another example of a substrate 108 printed with a repeating series of graphics (G1-G5) in the MD direction wherein each graphic illustrates a portion of a story. After graphic G5, the series may repeat again to illustrate the same story or may begin a series of graphics illustrating a different story, and so on.

As previously mentioned, components of the printing stations 104 may be located relatively close to the outer surface 106 of the central impression cylinder 102 so as to create nips 110 between the printing stations 104', 104", the substrate 108, and central impression cylinder 102. In particular, the print cylinders 112', 112" can be located relatively close to the central impression cylinder 102 in order to form a nip 110 between the printing plates 1000, (1001-100n) and the outer surface 106 of the central impression cylinder 102. In some embodiments, the printing stations 104 can be configured such that the distance between the printing plates and the central impression cylinder can be adjusted, which in turn, allows for adjustable nip pressures at each printing station. During operation of the printing apparatus, the substrate 108 is advanced into nips 110 between the central impression cylinder 102 and the printing stations 104', 104". As the substrate 108 passes through the nips 110, the nip pressures help maintain the substrate in a constant or fixed position relative to the outer surface 106 of central impression cylinder 102. As such, the nips help provide relatively precise and consistent print registration.

As mentioned above, printing apparatuses and methods according to the present disclosure can be configured to print a number, n, of graphics (G1-Gn) in the MD direction of a substrate 108. The printed substrate 108 can be cut into individual components and/or combined with other substrates or components or otherwise modified during the manufacture of absorbent articles. Examples of such printed substrates can be used in the manufacture of printed diaper components, such as for example, backsheets, topsheets, landing zones, fasteners, ears, absorbent cores, and acquisition layers. It is to be appreciated that different printed diaper components may require different MD lengths. Table 1 below provides example MD lengths for various diaper components for different size diapers, which may vary by about 1% on all sizes.

**Table 1**

| | Backsheet Outer Cover Film and Nonwoven Substrates | Topsheet Nonwoven and Liner Substrates | Landing Zone | Carton Board Container |
|---|---|---|---|---|
| Size 0 | 316 mm | 316 mm | 35 mm to 55 mm | 100 mm to 400 mm |
| Size 1 | 372 mm | 372 mm | | |
| Size 2 | 402 mm | 402 mm | | |
| Size 3 | 439 mm | 439 mm | | |
| Size 4 | 488 mm | 488 mm | | |
| Size 5 | 516 mm | 516 mm | | |
| Size 6 | 527 mm | 527 mm | | |
| Size 7 | 555 mm | 555 mm | | |
| Size 8 | 580 mm | 580 mm | | |
| Adult | 800 to 1000 mm | 800 to 1000 mm | | |

It is also to be appreciated that the printed graphic may not always define a length in the MD direction that is equal to the component length in the MD direction. However, as discussed below with reference to the example embodiment shown in Figs. 3A-3C, the printing apparatuses according the present disclosure can be configured to print various numbers of graphics on substrates used to manufacture various components wherein the MD lengths of the graphics are substantially equal to or less than the MD lengths of the individual components.

Fig. 2B shows detailed side view of a portion of a constant graphic printing station 104' wherein 2 printing plates 1000 are disposed on the print cylinder 112', and Fig. 2C shows a top side view of an embodiment of one printing plate 1000 shown in Fig. 2B. As shown in Fig. 2B, the printing plates 1000 each define a length in the MD direction, L_{PLATE}, and each of the printing plates may also be separated from each other in the MD direction by a distance, d. It is to be appreciated that d may be equal to or greater than zero. The sum of the length, L_{PLATE}, and the distance, d, defines a repeat length, L_{REPEAT}. L_{REPEAT} may also correspond to the length of substrate 108 in the MD direction that moves past a printing station 104' from the point at which a printing plate initially acts upon the substrate before a subsequent printing plate engages the substrate. As discussed above, the printing plates 1000 include identical printing patterns 2000 that transfer ink to the substrate 108 to print identical graphics IG thereon. As shown in Fig. 2C, the printing patterns 2000 may also define a length in the MD direction, L_{PATTARN}, which also corresponds to the length in the MD direction of the corresponding identical graphics IG printed on the substrate 108. It is to be appreciated that the constant graphic printing stations may include more or less than 2 printing plates. For example, in one embodiment the constant graphic printing station includes 1 printing plate disposed on the print cylinder, and in another embodiment, the constant graphic printing station includes 3 printing plates disposed on the print cylinder.

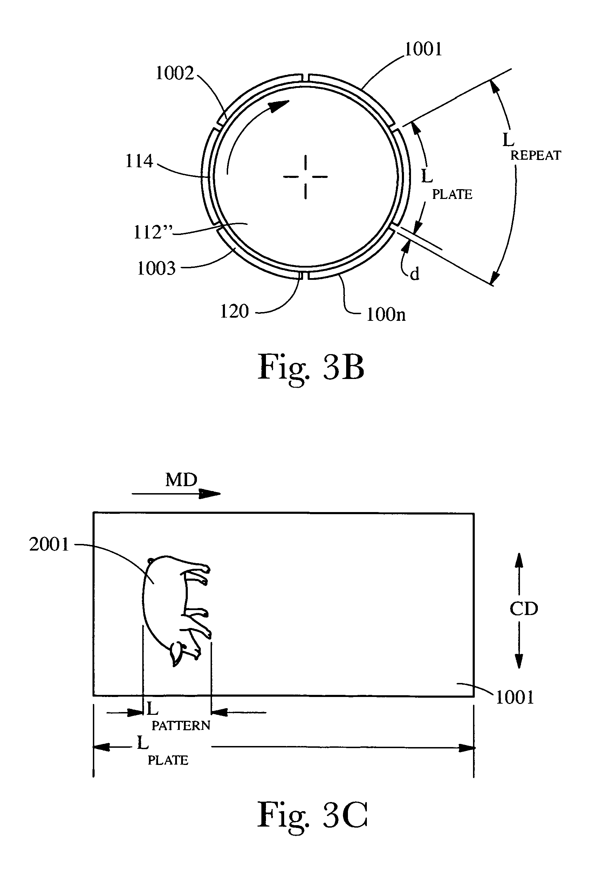

Fig. 3B shows detailed side view of a portion of a variable graphic printing station 104" wherein a plurality of printing plates (1001-100n) are disposed on the print cylinder 112", and Fig. 3C shows a top side view of an embodiment of one printing plate 1001 shown in Fig. 3B. As shown in Fig. 3B, the printing plates (1001-100n) each define a length in the MD direction, L_{PLATE}, and each of the printing plates may also be separated from each other in the MD direction by a distance, d. It is to be appreciated that d may be equal to or greater than zero. The sum of the length, L_{PLATE}, and the distance, d, defines a repeat length, L_{REPEAT}. L_{REPEAT} may also correspond to the length of substrate 108 in the MD direction that moves past a printing station 104" from the point at which a printing plate initially acts upon the substrate before a subsequent printing plate engages the substrate. As discussed above, the printing plates (1001-100n) include respective printing patterns (2001-200n) that transfer ink to the substrate 108 to print graphics (G1-Gn) thereon. As shown in Fig. 3C, the printing patterns (2001-200n) may also define a length in the MD direction, L_{PATTERN}, which also corresponds to the length in the MD direction of the corresponding graphics (G1-Gn) printed on the substrate 108. It is to be appreciated that the variable graphic printing stations may include more or less than 6 printing plates. For example, in one embodiment the constant graphic printing station includes 3 printing plate disposed on the print cylinder, and in another embodiment, the constant graphic printing station includes 8 printing plates disposed on the print cylinder.

The printing stations 104 can be configured to accommodate different values of L_{REPEAT} and L_{PATTERN}. In some embodiments, L_{PLATE} of the printing plates on the constant graphic print stations 104' may be equal to the L_{PLATE} of the printing plates on the variable graphic print stations 104". In another configuration, the constant graphic print stations 104' may define repeat length, L_{REPEAT}, that is equal to the repeat length, L_{REPEAT}, defined by the variable graphic print stations 104". In another configuration, the variable graphic print stations 104" may define repeat length, L_{REPEAT}, that is a multiple of the repeat length, L_{REPEAT}, defined by the constant graphic print stations 104'. For example, the repeat length, L_{REPEAT}, of the variable graphic print stations 104" may be 2 or 3 times the repeat length, L_{REPEAT}, of the constant graphic print stations 104'. In yet another configuration, the constant graphic print stations 104' may define repeat length, L_{REPEAT}, that is a multiple of the repeat length, L_{REPEAT}, defined by the variable graphic print stations 104". For example, the repeat length, L_{REPEAT}, of the constant graphic print stations 104' may be 2 or 3 times the repeat length, L_{REPEAT}, of the variable graphic print stations 104". In yet other examples, the repeat length of the constant graphic printing stations 104' and the variable graphic printing stations 104" may be configured to be substantially equal to the MD length of a printed component. More particularly, in embodiments configured to print graphics on a substrate used to manufacture printed backsheet or topsheet components, L_{REPEAT} of both the constant graphic printing stations 104' and the variable graphic printing stations 104" may be equal to or substantially equal to the MD length of an individual backsheet or topsheet, and as such, in some embodiments, the L_{REPEAT} may correspond with the MD length values provided in Table 1 above. For example, embodiments configured to print graphics on a substrate used to manufacture printed backsheets and/or topsheets for diapers, L_{REPEAT} of both the constant graphic printing stations 104' and the variable graphic printing stations 104" may be equal to or substantially equal to 316 mm to 1000 mm, depending on the diaper size. In another embodiment configured to print graphics on a substrate used to manufacture printed landing zones, L_{REPEAT} of both the constant graphic printing stations 104' and the variable graphic printing stations 104" may be equal to or substantially equal to the MD length of an individual landing zone, and as such, in some embodiments, the L_{REPEAT} may be equal to or substantially equal to 35 mm to 55 mm.

It should also be appreciated that in some embodiments L_{PATTERN} may be equal to L_{REPEAT}, and in other embodiments, the L_{PATTERN} may be less than L_{REPEAT}. As such, MD length defined by printed graphics may span the entire MD length of a printed component or may span a portion of the entire MD length of a printed component. It should also be appreciated that the patterns 2000, (2001-200n) may be located in different positions along the MD and/or CD directions of the printing plates 1000, (1001-100n). As such, graphics can be located in different positions along the MD length and CD width of an absorbent article component. For example, a graphic may be located in front or back waist regions or a crotch region of a backsheet or topsheet as well as being right, left, or centrally oriented relative to the CD direction. In other examples, a graphic may span the entire length of a backsheet or topsheet, such as from a front waist region to a back waist region. It should further be appreciated that one or more printing plates 1000, (1000-100n) may include more than one printing pattern 2000, (2001-200n). Thus, a plurality of graphics can be located in different positions along the MD length and CD width of an absorbent article component.

The number, n, of graphics printed in a series on a substrate may be increased or decreased by increasing or decreasing, respectively, the number n of printing plates and associated printing patterns mounted on the print cylinders. For example, some embodiments of printing apparatuses may include variable graphic printing stations 104" each having 2 or more printing plates, and some embodiments may include printing stations each having 10 or more printing plates in the MD direction. As such, for a given L_{REPEAT}, a relatively larger diameter print cylinder 112" may be required to accommodate relatively higher numbers of printing plates. Conversely, for a given L_{REPEAT}, a relatively smaller diameter print cylinder 112" may be required to accommodate relatively lower numbers of printing plates. Table 2 below illustrates examples of n graphics that may be printed in a series for various diaper components:

**Table 2**

| Absorbent Article Component | Example Values of Lrepeat | Numbers of Different Graphics in a Series, n |
|---|---|---|
| Backsheet | 316 mm to 1000 mm | 2 to 9 |
| Topsheet | 316 mm to 1000 mm | 2 to 9 |
| Landing Zone | 35 mm to 55 mm | 2 to 175 |

With regard to the values provided in Table 2 above, it is to be appreciated that n can be greater than 12 and 340 depending on the value of L_{REPEAT} and the printing apparatus and print cylinder configuration. In addition, the L_{REPEAT} values in Table 2 may also be from about 316 mm to about 1000 mm and from about 35 mm to about 55 mm. As such, the example values provided in Table 2 illustrate that in some embodiments, n backsheets and topsheets having a L_{REPEAT} value of 316 mm or about 316 mm may be printed with series of n graphics, wherein n can be from 2 to 12 (or greater than 12), as well any value in between, such as 5 or 10. Similarly, n backsheets and topsheets having a L_{REPEAT} value of 1000 mm or about 1000 mm may be printed with series of n graphics, wherein n can be from 2 to 12 (or greater than 12), as well any value in between, such as for example, 5 or 10.

Further yet, n landing zones having a L_{REPEAT} value of 35 mm or about 35 mm may be printed with series of n graphics, wherein n can be from 2 to 340 (or greater than 340), as well any value in between. Similarly, n landing zones having a L_{REPEAT} value of 55 mm or about 55 mm may be printed with series of n graphics, wherein n can be from 2 to 340 (or greater than 340), as well any value in between.

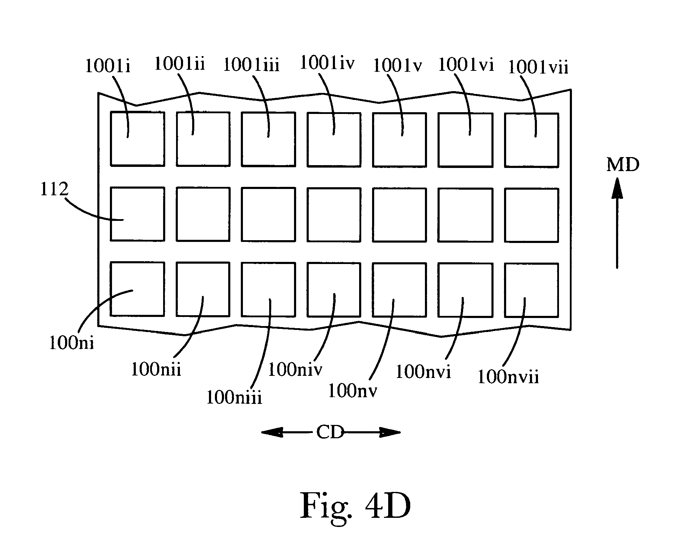

It should also be appreciated that the embodiments of the printing apparatuses can be configured with various CD widths. For example, in some embodiments, the CD width may be 6 inches. In other embodiments, the CD width may be 64 inches. In still other embodiments, the CD width may be 100 inches. It should also be appreciated that the printing stations can also be configured to include various numbers and sizes of printing plates oriented along the CD width of the print cylinder. For example, some embodiments can be configured with 5, 7, 9, or more printing plates along the CD width of the print cylinder. Fig. 4D illustrates a portion of an embodiment of a print cylinder 112" configured with seven printing plates (1001i-1001vii) disposed along the CD width of the print cylinder 112", and n printing plates arranged along the MD direction of the print cylinder 112". In other words, the print cylinder shown in Fig. 4D has seven lanes of printing plates disposed along the CD direction, wherein each lane includes n printing plates. Thus, depending on a particular configuration, the printing plates shown in Fig. 4D can print seven identical or different series of n graphics in the MD direction of a substrate, wherein each series of n graphics are arranged along the CD width of the substrate. It is to be appreciated that although Fig. 4D shows seven printing plates or lanes arranged in the CD direction, more or less than seven printing plates or lanes can be arranged in the CD direction.

As previously mentioned, embodiments of the printing apparatus can be configured to include various numbers of printing stations 104. For example, as shown in Fig. 2, the printing apparatus 100 includes four constant graphic printing stations 104' and four variable graphic printing stations 104". In addition, the printing stations may utilize different types of ink as well as different colors.

In one example, the printing apparatus may be configured with one constant graphic printing station 104' that is configured to repeatedly print identical graphics on the substrate. In another example, the printing apparatus may be configured with four constant graphic printing stations CMYK printing wherein a first printing station 104'a is adapted to print cyan, a second printing station 104'b is adapted to print magenta, a third printing station 104'c is adapted to print yellow, and a fourth printing station 104'd is adapted to print black. In yet another example, each constant graphic printing station 104' may be configured such that each station repeatedly prints an identical graphic wherein the identical graphics printed by each constant graphic print station are different from the identical graphics printed by the other constant graphic print stations.

In other examples, the variable graphic printing stations 104" may be configured for CMYK printing wherein a first printing station 104"a is adapted to print cyan, a second printing station 104"b is adapted to print magenta, a third printing station 104"c is adapted to print yellow, and a fourth printing station 104"d is adapted to print black. The different ink colors and types may be used in combination to print an entire graphic on the substrate. In some configurations, a single printing station may be used to print a unitary color graphic on the substrate.

The printing stations 104 may also be configured to print graphics on a substrate that may appear in a relatively large range of colors through various different processes, such as for example, halftone printing. Halftone printing utilizes equally spaced dots of ink to simulate a continuous tone. Various descriptions of halftone printing processes are discloses in U.S. Patent Nos. 4,142,462; 5,205,211; 5,617,790; 7,126,724; as well as U.S. Patent Publication No. 20040160644 and PCT Publication No. WO98/06006A1, which are all incorporated by reference herein.

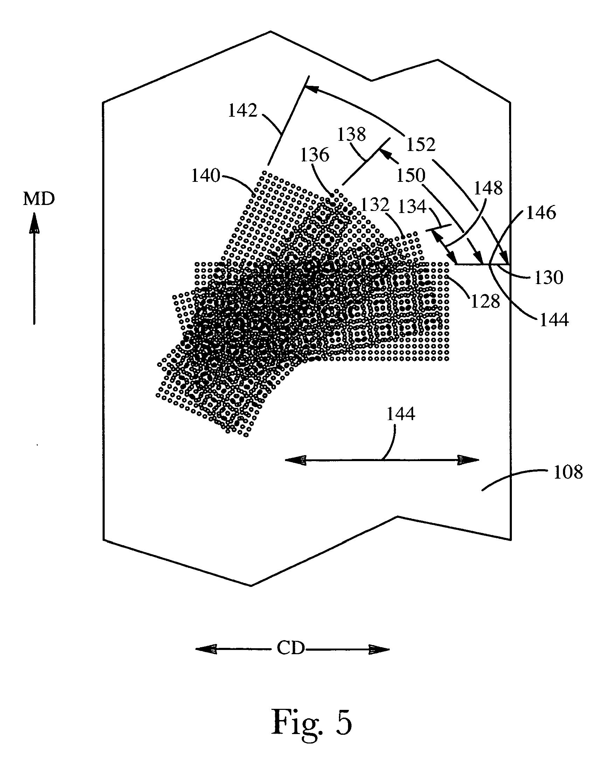

In some embodiments, the constant graphic printing stations 104' and/or the variable graphic printing stations 104" may be configured for halftone printing. For example, the first, second, third, and fourth variable graphic printing stations (104"a-104"d) may be configured to print cyan; magenta, yellow, and black colors, respectively. More particularly, the printing plates (1001-100n) on each printing station 104" are configured to print dots of ink of each respective color on the substrate. In addition, the printing plates (1001-100n) may be configured to print dots of various shapes, such as for example, round, elliptical, or square. Each printing station (104"a-104"d) may also be configured to print the dots in rows that extend along and/or parallel to respective axes. For example, with reference to Figs. 1 and 5, the first printing station 104"a may print rows of first color (e.g. cyan) dots 128 along or parallel to a first axis 130, the second printing station 104"b may print rows of second color (e.g. magenta) dots 132 along or parallel to a second axis 134, the third print station 104"c may print rows of third color (e.g. yellow) dots 136 along or parallel to a third axis 138, and the fourth print station 104"d may print rows of fourth color (e.g. black) dots 140 along or parallel to a fourth axis 142.

In halftone printing, the dot axes may be oriented at different angles, which may be referred to as screen angles, relative to a reference axis 144. As shown in Fig. 5, the first axis 130 may define a first screen angle 146, the second axis 134 may define a second screen angle 148, the third axis 136 may define a third screen angle 150, and the fourth axis 142 may define a fourth screen angle 152 relative to a reference axis. It is to be appreciated that various reference axes may be used as a basis for defining the screen angles. For example, the reference axis 144 shown in Fig. 5 is oriented in the CD direction on the substrate and is parallel to the first axis 130. In another example, the reference axis 144 may be oriented in the MD direction. In other examples, the reference angle may be parallel to any of the dot print axes. The resolution of halftone printing can be measured in lines per inch (lpi), which corresponds to the number of lines of dots in one inch as measured along a screen angle. It is to be appreciated that the printing apparatus can be configured to print various resolutions. For example, some embodiments can be configured to print line densities in the range of about 45 lpi to about 185 lpi. It should also be appreciated that the printing apparatus can be configured to print dots at various screen angles. For example, the table below illustrates six examples of screen angles that may be used:

**Table 3**

| Ink Color | Example 1 Screen Angles | Example 2 Screen Angles | Example 3 Screen Angles | Example 4 Screen Angles | Example 5 Screen Angles | Example 6 Screen Angles |
|---|---|---|---|---|---|---|
| Cyan | 112° | 105° | 15° | 75° | 105° | 15° |
| Magenta | 82° | 75° | 75° | 15° | 75° | 45° |
| Yellow | 97° | 0° or 90° | 0° or 90° | 0° | 90° | 0° |
| Black | 52° | 45° | 45° | 45° | 15° | 75° |

In operation, the printing stations print dots at predetermined screen angles to produce graphics having desired colors. The dots printed by the printing stations may also be overlayed and may produce a pattern. In one example, the patterns may form a plurality of rosettes. In one embodiment, the printing stations are configured to produce open rosettes. In another embodiment, the printing stations are configured to produce closed rosettes. The dots may also be printed such that portions of subsequently printed dots overlap portions of previously printed dots to produce desired color combinations.

As discussed above, the variable graphic printing stations 104" may be configured with n printing plates (1001-100n), wherein n may be 2 or greater and wherein the printing stations are configured to print different colors of ink. For the purposes of illustration with reference to the printing apparatus 100 shown in Fig. 1, the first variable graphic print station 104"a may be configured with n printing plates 1001a to 100na; the second print station 104"b may be configured with n printing plates 1001b to 100nb; the third print station 104"c may be configured with n printing plates 1001c to 100nc; and the fourth print station 104"d may be configured with n printing plates 1001d to 100nd. In addition, the first printing station 104"a may be configured to print a first color ink at a first screen angle 146; the second printing station 104"b may be configured to print a second color ink at a second screen angle 148; the third printing station 104"c may be configured to print a third color ink at a third screen angle 150; and the fourth printing station 104"d may be configured to print a fourth color ink at a fourth screen angle 152. In some embodiments, the screen angles may be in accordance with those provided in Table 3. In addition, depending on the desired color combinations, portions of the some dots of ink printed by printing stations may be printed to overlap portions of some dots of ink printed by other printing stations. For example, the second printing station 104"b may print dots of ink that overlap portions of dots of ink printed by first printing station 104"a. In addition, the third printing station 104"c may print dots of ink that overlap portions of dots of ink printed by the second printing station 104"b and/or the first printing station 104"a. Further, the fourth printing station 104"d may print dots of ink that overlap portions of dots of ink printed by the third printing station 104"c, the second printing station 104"b, and/or the first printing station 104"a.

With reference to the apparatus 100 of Fig. 1, during operation, the substrate 108 on the rotating central impression cylinder 102 moves past the constant graphic printing stations (104'a-104'd) and the variable graphic printing stations (104"a-104"d). Printing plates 1000 from the constant graphic printing stations print ink on the substrate 108 to form a series of identical graphics IG. For example, Fig. 4A shows a series of identical graphics IG printed on the substrate. The constant graphic printing stations (104'a-104'd) may be configured to print the same graphic ID with different ink colors, such as in a CMYK configuration. For example, the identical graphic IG shown in Fig. 4A is printed: with a first ink color by the first constant graphic printing station 104'a; with a second ink color by the second constant graphic printing station 104'b; with a third ink color by the third constant graphic printing station 104'c; and with a fourth ink color by the fourth constant graphic printing station 104'd. It is to be appreciated that although the constant graphic printing stations 104' are shown with reference to Fig. 4A to each repeatedly print an identical graphic that is different from the identical graphics of the other three constant graphic printing stations, the constant graphic printing stations 104' can be configured to print the different graphics with different or same ink colors, such as in a spot color configuration.

With further reference to Fig. 4B, during operation of the apparatus in Fig. 1, printing plates 1001a, 1001b, 1001c, and 1001d from the variable graphic printing stations (104"a-104"d) print ink on the substrate 108 to form a first graphic G1 on the substrate adjacent the identical graphics IG. In conjunction with the rotation of the central impression cylinder 102 and coordinated advancement of the print cylinders 112 on the printing stations (104"a-104"d), printing plates 1002a, 1002b, 1002c, and 1002d print ink on the substrate to form a second graphic G2 on the substrate 108, wherein the first graphic G1 is downstream of the second graphic G2 in the MD direction as shown in Fig. 4B. The process continues to advance printing plates 1003a, 1003b, 1003c, and 1003d to plates 100na, 100nb, 100nc, and 100nd resulting in a series of n graphics (G1-Gn) being printed on the substrate 108 in the MD direction. Once the nth graphic, Gn, is printed, the process is continuously repeated beginning again with plates 1001a, 1001b, 1001c, and 1001d through plates 100na, 100nb, 100nc, and 100nd, resulting in the series of n graphics (G1-Gn) being repeated along MD direction along a length of the substrate 108. As previously mentioned, the graphics G1 through Gn may be different from each other. Fig. 4B shows an example of a repeating series of 6 different graphics G1 through G6 printed adjacent the identical graphics IG. And Fig. 4C shows an example of a repeating series of 5 different graphics G1 through G5 printed adjacent the identical graphics IG.

The printing apparatuses disclosed herein may also be configured to print at various speeds. For example, embodiments may be configured to print graphics on a substrate that allows the substrate to advance in the MD direction at a speed that is substantially equal to a converting process speed, which may be defined by a number of pads or absorbent articles per minute. For example, when used in conjunction with a diaper converting process producing 800 diapers per minute, a printing apparatus may be configured to correspondingly print 800 graphics per minute on the substrate. In other examples, the printing apparatus may be configured to print at speed of greater than 800 graphics per minute. In another example, the embodiments of the printing apparatus may be configured to print graphics on a substrate that allows that substrate to advance in the MD direction at a speed that may be defined by a number of feet or meters of substrate per minute. In some examples, the printing apparatus may be configured to print at a speed of greater than 800 meters per minute.

As discussed above with reference to Fig. 3F, the printing apparatus may be configured with more than one printing plate in the CD direction so as to have a plurality of lanes of printing plates. Having additional lanes of printing plates in CD direction may increase the printing capacity or throughput of the printing apparatus.

For example, some embodiments may be configured print cylinders having multiple lanes of printing plates in the CD direction, and wherein the print cylinders are from 12 inches CD width to 100 or 200 inches CD width, which could accommodate manufacturing line speeds of about 300 to 2000 feet per minute.

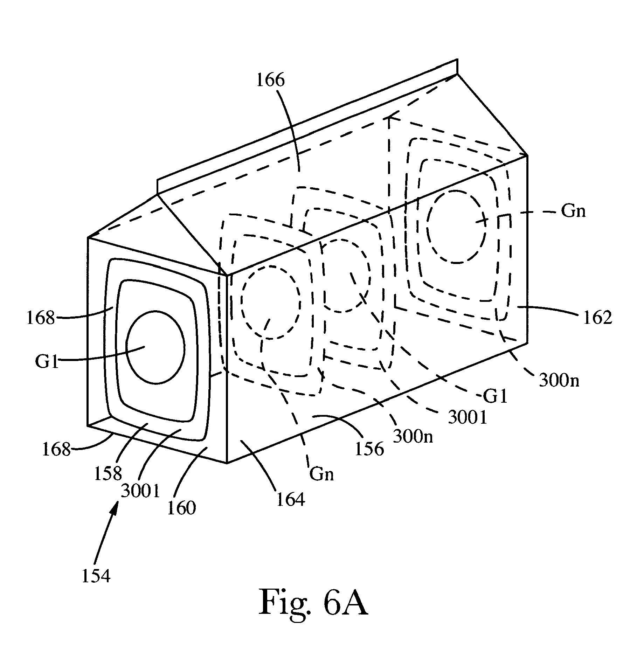

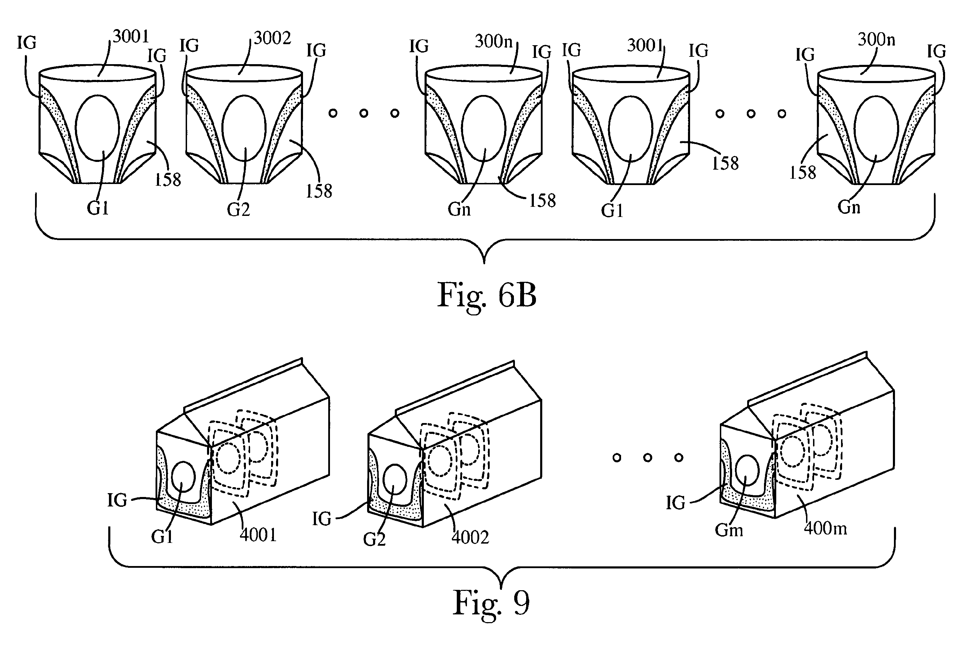

As discussed above, the printing apparatuses and processes disclosed herein may be used to print graphics on substrates used to produce absorbent articles, such as diapers. In addition, an absorbent product may be produced by placing the absorbent articles in a package. For example, Fig. 6A shows a perspective view of an absorbent product 154 constructed in accordance with the methods and apparatuses of the present disclosure. As shown in Fig. 6A, the absorbent product 154 includes a package 156 and a plurality of absorbent articles 158 which are stacked and contained in the package 156. As discussed above, the absorbent articles 158 may include printed components made from substrates printed with the printing apparatuses and methods disclosed herein. As discussed below with reference to absorbent articles in the form of diapers, examples of such printed components, may include for example, backsheets, topsheets, landing zones, fasteners, ears, absorbent cores, and acquisition layers. As mentioned above, the printed components may also be constructed from a substrate 108 having a repeating series of n graphics (G1-Gn), wherein each of the n graphics may be different from each other. Once the desired components are assembled and separated into discrete absorbent articles 158, such as diapers, an absorbent product may be manufactured by folding, stacking, and placing one or more, or a portion of, the series of n absorbent articles in a package. As shown in Figs. 6A and 6B, n adjacent absorbent articles (3001-300n) having different graphics (G1-Gn) printed thereon are contained in the package. It is to be appreciated that the absorbent product may include various numbers of absorbent articles. For example, the package may contain absorbent articles with the more or less than one repeating series of graphics.

It is to be appreciated that the package 156 may have various types of shapes and sizes. As shown for example in Fig. 6A, the package 156 may include a front panel 160, a rear panel 162 opposed to the front panel 160, side panels 164 connected with the front and rear panels, a top gusset panel 166 connected with the front, rear, and side panels, and a bottom panel 168 opposed to the top panel 166. Each of the front and rear panels, the side panels, and the bottom panel may also be substantially planar as shown in Fig. 6A. The package 156 may also include a transparent window allows at least one of the variable graphics G1-Gn and/or identical graphics IG to be viewed from outside the package. It should be appreciated that the package may include windows of various sizes and shapes located on various parts of the package. As shown in Fig. 6A, the transparent window 168 is located on the front panel 160, which shows the graphic G1 printed on the absorbent article 158. It is to also be appreciated that the package can be constructed from various types of materials. For example, the package may be in the form of a carton made from a cardboard material. In other examples, the package may be in the form of a flexible bag made from a thin film material, such as for example, paper, plastic, recyclable material, or laminate material comprised of two or more the thin film materials. In one embodiment, the package is in the form of a poly bag made from a polyethylene film.

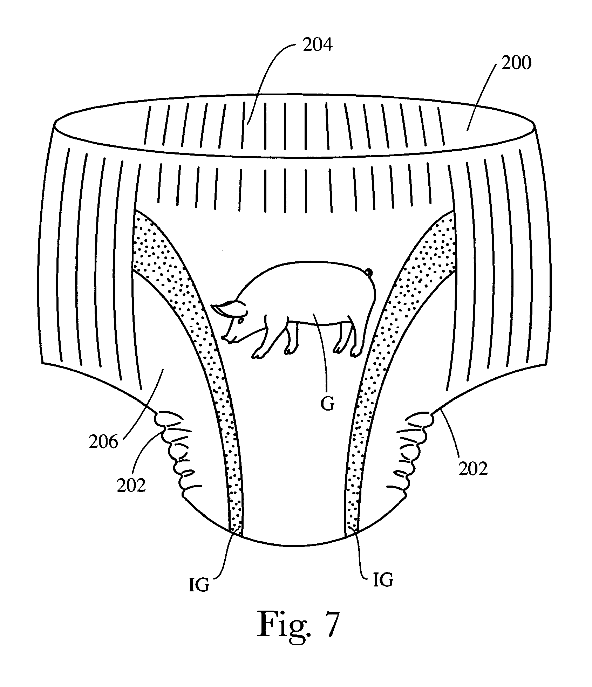

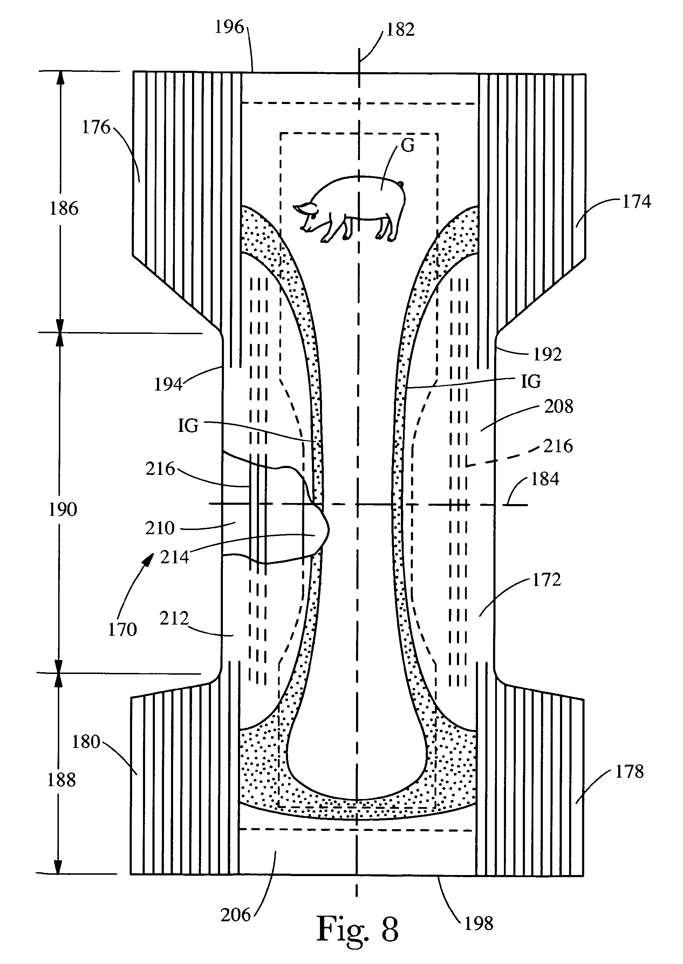

The absorbent product 154 may also include various types of absorbent articles 158. For example, the absorbent product shown in Fig. 6A includes a plurality of diapers. As mentioned above, the diapers (3001-300n) may include printed components with repeating series of different graphics (G1-Gn) and identical graphics IG printed thereon. For the purposes of a specific illustration, Fig. 7 shows one example of a disposable absorbent article in the form of a diaper 170 which may be contained in the package shown in Figs. 6A-6B. Fig. 8 is a plan view of the diaper 170 including a chassis 172 shown in a flat, unfolded condition, with the portion of the diaper that faces away from a wearer oriented towards the viewer. A portion of the chassis structure is cut-away in Fig. 8 to more clearly show the construction of and various features that may be included in embodiments of the diaper.

As shown in Figs. 8, the diaper 170 includes a 172 chassis having a first ear 174, a second ear 176, a third ear 178, and a fourth ear 180. To provide a frame of reference for the present discussion, the chassis 172 is shown with a longitudinal axis 182 and a lateral axis 184. The chassis 172 is shown as having a first waist region 186, a second waist region 188, and a crotch region 190 disposed intermediate the first and second waist regions. The periphery of the diaper is defined by a pair of longitudinally extending side edges 192, 194; a first outer edge 196 extending laterally adjacent the first waist region 186; and a second outer edge 198 extending laterally adjacent the second waist region 188. As shown in Fig. 7, the diaper 170 has a waist opening 200 and two leg openings 202. The diaper 170 may also be provided in the form of a pant-type diaper or may alternatively be provided with a re-closable fastening system, which may include fastener elements in various locations to help secure the diaper in position on the wearer. For example, fastener elements may be located on the ears and may be adapted to releasably connect with one or more corresponding fastening elements located in the first or second waist regions.

As shown in Figs. 7 and 8, the chassis includes an inner, body facing surface 204, and an outer, garment facing surface 206. As shown in Fig. 8, the chassis 172 may include an outer covering layer 208 including a topsheet 210 and a backsheet 212. An absorbent core 214 may be disposed between a portion of the topsheet 210 and the backsheet 212. It is to be appreciated that any one or more of the regions of the chassis may be stretchable and may include various types of elastomeric materials and/or laminates. As such, the diaper may be configured to adapt to a specific wearer's anatomy upon application and to maintain coordination with the wearer's anatomy during wear.

Embodiments of the diaper may also include pockets for receiving and containing waste, spacers which provide voids for waste, barriers for limiting the movement of waste in the article, compartments or voids which accept and contain waste materials deposited in the diaper, and the like, or any combinations thereof. Examples of pockets and spacers for use in absorbent products are described in U.S. Pat. No. 5,514,121 issued to Roe et al. on May 7, 1996, entitled "Diaper Having Expulsive Spacer"; U.S. Pat. No. 5,171,236 issued to Dreier et al on Dec. 15, 1992, entitled "Disposable Absorbent Article Having Core Spacers"; U.S. Pat. No. 5,397,318 issued to Dreier on Mar. 14, 1995, entitled "Absorbent Article Having A Pocket Cuff"; U.S. Pat. No. 5,540,671 issued to Dreier on Jul. 30, 1996, entitled "Absorbent Article Having A Pocket Cuff With An Apex"; and PCT Application WO 93/25172 published Dec. 3, 1993, entitled "Spacers For Use In Hygienic Absorbent Articles And Disposable Absorbent Articles Having Such Spacer"; and U.S. Pat. No. 5,306,266, entitled "Flexible Spacers For Use In Disposable Absorbent Articles", issued to Freeland on Apr. 26, 1994, which are all hereby incorporated by reference herein. Examples of compartments or voids are disclosed in U.S. Pat. No. 4,968,312, entitled "Disposable Fecal Compartmenting Diaper", issued to Khan on Nov. 6, 1990; U.S. Pat. No. 4,990,147, entitled "Absorbent Article With Elastic Liner For Waste Material Isolation", issued to Freeland on Feb. 5, 1991; U.S. Pat. No. 5,062,840, entitled "Disposable Diapers", issued to Holt et al on Nov. 5, 1991; U.S. Pat. No. 6,482,191 entitled "Elasticated Topsheet with an Elongate Slit Opening," issued to Roe et al. on Nov. 19, 2002; and U.S. Pat. No. 5,269,755 entitled "Trisection Topsheets For Disposable Absorbent Articles And Disposable Absorbent Articles Having Such Trisection Topsheets", issued to Freeland et al. on Dec. 14, 1993, which are all hereby incorporated by reference herein. Examples of suitable transverse barriers are described in U.S. Pat. No. 5,554,142 entitled "Absorbent Article Having Multiple Effective Height Transverse Partition" issued Sep. 10, 1996 in the name of Dreier et al.; PCT Patent WO 94/14395 entitled "Absorbent Article Having An Upstanding Transverse Partition" published Jul. 7, 1994 in the name of Freeland, et al., and U.S. Pat No. 5,653,703 Absorbent Article Having Angular Upstanding Transverse Partition, issued Aug. 5, 1997 to Roe, et al., which are all hereby incorporated by reference herein. All of the above-cited references are hereby incorporated by reference herein. In addition to or in place of the voids, pockets and barriers, described above, embodiments of the absorbent article may also include a waste management element capable of effectively and efficiently accepting, storing and/or immobilizing viscous fluid bodily waste, such as runny feces, such as described in U.S. Pat. No. 6,010,491 issued to Roe et al. on Jan. 4, 2000, which is hereby incorporated by reference herein.

As previously mentioned, the chassis 172 may include the backsheet 212, shown for example, in Fig. 8. In some embodiments, the backsheet is configured to prevent exudates absorbed and contained within the chassis from soiling articles that may contact the diaper, such as bedsheets and undergarments. Some embodiments of the backsheet may be fluid permeable, while other embodiments may be impervious to liquids (e.g., urine) and comprises a film, such as a thin plastic film. In some embodiments, the plastic film includes a thermoplastic film having a thickness of about 0.012 mm (0.5 mil) to about 0.051 mm (2.0 mils). Some backsheet films may include those manufactured by Tredegar Industries Inc. of Terre Haute, Ind. and sold under the trade names X15306, X10962, and X10964. Other backsheet materials may include breathable materials that permit vapors to escape from the diaper while still preventing exudates from passing through the backsheet. Exemplary breathable materials may include materials such as woven webs, nonwoven webs, composite materials such as film-coated nonwoven webs, and microporous films such as manufactured by Mitsui Toatsu Co., of Japan under the designation ESPOIR NO and by EXXON Chemical Co., of Bay City, Tex., under the designation EXXAIRE. Suitable breathable composite materials comprising polymer blends are available from Clopay Corporation, Cincinnati, Ohio under the name HYTREL blend P18-3097. Such breathable composite materials are described in greater detail in PCT Application No. WO 95/16746, published on Jun. 22, 1995 in the name of E. I. DuPont and U.S. Pat. No. 5,865,823, issued on Feb. 2, 1999 to Curro, both of which are hereby incorporated by reference herein. Other breathable backsheets including nonwoven webs and apertured formed films are described in U.S. Pat. No. 5,571,096 issued to Dobrin et al. on Nov. 5, 1996; and U.S. Pat. No. 6,573, 423 issued to Herrlein et al. on June 3, 2003, which are all hereby incorporated by reference herein.

The backsheet 212 may be formed by only one sheet (or layer) material such as a breathable (or microporous) film material or a non-breathable (or non-microporous) film material. In some embodiments, the backsheet may be formed by two (or more) sheet (or layer) materials which may include a non-breathable (or breathable) film material and a nonwoven outer cover material. In some embodiments, the backsheet may be formed by a laminate of two sheet (or layer) materials joined together, for example, the backsheet may include a non-breathable film material and a nonwoven material which is joined to the garment facing surface of the film material to provide a cloth-like and/or garment-like feel. In accordance with the discussion above, graphics may be printed on a substrate to make printed component material, which may be converted into printed components to manufacture the backsheet. Thus, the substrate may be in the form of a film material and/or nonwoven material used to construct the backsheet. As such, variable graphics G and/or identical graphics IG may be printed on any surface of the component material(s) of the backsheet. For example, graphics can be printed on any of the garment facing surfaces and the body facing surfaces of the film material and the nonwoven material. In some embodiments, graphics are printed directly on the nonwoven material. In other embodiments, the variable graphic G and identical graphic IG are printed on the garment facing surface of the film material. In such an arrangement, graphics may be covered (or protected) by the nonwoven material, wherein the graphics are visible through the nonwoven material.

As with the backsheet 212, graphics may be printed on a substrate used as a printed component material to construct the topsheet 210. As such, variable graphics G and/or identical graphics IG may be printed on any surface of the component material(s) of the topsheet. The topsheet may be constructed to be compliant, soft feeling, and non-irritating to the wearer's skin. Further, all or at least a portion of the topsheet may be liquid pervious, permitting liquid to readily penetrate therethrough. As such, the topsheet may be manufactured from a wide range of materials, such as porous foams; reticulated foams; apertured nonwovens or plastic films; or woven or nonwoven webs of natural fibers (e.g., wood or cotton fibers), synthetic fibers (e.g., polyester, polyethylene, or polypropylene fibers), or a combination of natural and synthetic fibers. If the absorbent assemblies include fibers, the fibers may be spunbonded, carded, wetlaid, meltblown, hydroentangled, or otherwise processed as is known in the art. One example of a topsheet including a web of staple length polypropylene fibers is manufactured by Veratec, Inc., a Division of International Paper Company, of Walpole, Mass. under the designation P-8.

Examples of formed film topsheets are described in U.S. Pat. No. 3,929,135, entitled "Absorptive Structures Having Tapered Capillaries," which issued to Thompson on Dec. 30, 1975; U.S. Pat. No. 4,324,246, entitled "Disposable Absorbent Article Having A Stain Resistant Topsheet," which issued to Mullane, et al. on Apr. 13, 1982; U.S. Pat. No. 4,342,314, entitled "Resilient Plastic Web Exhibiting Fiber-Like Properties," which issued to Radel, et al. on Aug. 3, 1982; U.S. Pat. No. 4,463,045, entitled "Macroscopically Expanded Three-Dimensional Plastic Web Exhibiting Non-Glossy Visible Surface and Cloth-Like Tactile Impression," which issued to Ahr, et al. on Jul. 31, 1984; and U.S. Pat. No. 5,006,394, entitled "Multilayer Polymeric Film," which issued to Baird on Apr. 9, 1991, all of which are hereby incorporated by reference herein. Other topsheets may be made in accordance with U.S. Pat. Nos. 4,609,518 and 4,629,643, which issued to Curro et al. on Sep. 2, 1986, and Dec. 16, 1986, respectively, both of which are hereby incorporated by reference herein. Such formed films are available from The Procter & Gamble Company of Cincinnati, Ohio as "DRI-WEAVE" and from Tredegar Corporation of Terre Haute, Ind. as "CLIFF-T."

In some embodiments, the topsheet is made of a hydrophobic material or is treated to be hydrophobic in order to isolate the wearer's skin from liquids contained in the absorbent core. If the topsheet is made of a hydrophobic material, at least the upper surface of the topsheet may be treated to be hydrophilic so that liquids will transfer through the topsheet more rapidly. This diminishes the likelihood that body exudates will flow off the topsheet rather than being drawn through the topsheet and being absorbed by the absorbent core. The topsheet can be rendered hydrophilic by treating it with a surfactant or by incorporating a surfactant into the topsheet. Suitable methods for treating the topsheet with a surfactant include spraying the topsheet material with the surfactant and immersing the material into the surfactant. A more detailed discussion of such a treatment and hydrophilicity is contained in U.S. Pat. No. 4,988,344, entitled "Absorbent Articles with Multiple Layer Absorbent Layers," which issued to Reising, et al. on Jan. 29, 1991, and U.S. Pat. No. 4,988,345, entitled "Absorbent Articles with Rapid Acquiring Absorbent Cores," which issued to Reising on Jan. 29, 1991, all of which are hereby incorporated by reference herein. A more detailed discussion of some methods for incorporating surfactant in the topsheet can be found in U.S. Statutory Invention Registration No. H1670, which was published on Jul. 1, 1997, in the names of Aziz et al., all of which are hereby incorporated by reference herein.

In some embodiments, the topsheet may include an apertured web or film that is hydrophobic. This may be accomplished eliminating the hydrophilizing treatment step from the production process and/or applying a hydrophobic treatment to the topsheet, such as a polytetrafluoroethylene compound like SCOTCHGUARD or a hydrophobic lotion composition, as described below. In such embodiments, the apertures may be large enough to allow the penetration of aqueous fluids like urine without significant resistance. A more detailed discussion of various apertured topsheets can be found in U.S. Pat. No. 5,342,338, entitled "Disposable Absorbent Article for Low-Viscosity Fecal Material," which issued to Roe on Aug. 30, 1994; U.S. Pat. No. 5,941,864, entitled "Disposable Absorbent Article having Improved Fecal Storage," which issued to Roe on Aug. 24, 1999; U.S. Pat. No. 6,010,491, entitled "Viscous Fluid Bodily Waste Management Article," which issued to Roe et al. on Jan. 4, 2000; and U.S. Pat. No. 6,414,215, entitled "Disposable Absorbent Article having Capacity to Store Low-Viscosity Fecal Material," which issued to Roe on July 2, 20002, all of which are hereby incorporated by referenced herein.

Any portion of the topsheet may be coated with a lotion, such as topsheets described in U.S. Pat. No. 5,607,760, entitled "Disposable Absorbent Article Having A Lotioned Topsheet Containing an Emollient and a Polyol Polyester Immobilizing Agent," which issued to Roe on Mar. 4, 1997; U.S. Pat. No. 5,609,587, entitled "Diaper Having A Lotion Topsheet Comprising A Liquid Polyol Polyester Emollient And An Immobilizing Agent," which issued to Roe on Mar. 11, 1997; U.S. Pat. No. 5,635,191, entitled "Diaper Having A Lotioned Topsheet Containing A Polysiloxane Emollient," which issued to Roe et al. on Jun. 3, 1997; U.S. Pat. No. 5,643,588, entitled "Diaper Having A Lotioned Topsheet," which issued to Roe et al. on Jul. 1, 1997; and U.S. Pat. No. 6,498,284, entitled "Disposable Absorbent Article with a Skin Care Composition on an Apertured Top Sheet," which issued to Roe on Dec. 24, 2002, all of which are hereby incorporated by reference herein. The lotion may function alone or in combination with another agent as the hydrophobizing treatment described above. The topsheet may also include or be treated with antibacterial agents, some examples of which are disclosed in PCT Publication No. WO 95/24173 entitled "Absorbent Articles Containing Antibacterial Agents in the Topsheet For Odor Control," which was published on Sep. 14, 1995, in the name of Theresa Johnson, which is hereby incorporated by reference herein. Further, the topsheet, the backsheet, or any portion of the topsheet or backsheet may be embossed and/or matte finished to provide a more cloth like appearance.

The absorbent core 214 may include components such as an acquisition layer and absorbent material that is generally compressible, conformable, non-irritating to the wearer's skin, and capable of absorbing and retaining liquids such as urine and other body exudates. Thus, in addition to backsheet and topsheet components, it should be appreciated that graphics may be printed on substrates used as printed component material to construct the absorbent core and acquisition layer. In addition, variable graphics G and/or identical graphics IG may be printed on any surface of various component material(s) of the absorbent core. The absorbent core can also be manufactured in a wide variety of sizes and shapes (e.g., rectangular, hourglass, T-shaped, asymmetric, etc.). The absorbent core may also include a wide variety of liquid-absorbent materials commonly used in disposable diapers and other absorbent articles. In one example, the absorbent core includes comminuted wood pulp, which is generally referred to as airfelt. Examples of other absorbent materials include creped cellulose wadding; meltblown polymers, including coform; chemically stiffened, modified or cross-linked cellulosic fibers; tissue, including tissue wraps and tissue laminates; absorbent foams; absorbent sponges; superabsorbent polymers; absorbent gelling materials; or any other known absorbent material or combinations of materials.

It is to be appreciated that the configuration and construction of the absorbent core may be varied (e.g., the absorbent core(s) or other absorbent structure(s) may have varying caliper zones, a hydrophilic gradient, a superabsorbent gradient, or lower average density and lower average basis weight acquisition zones; or may comprise one or more layers or structures).

Exemplary absorbent structures are described in U.S. Pat. No. 4,610,678, entitled "High-Density Absorbent Structures," which issued to Weisman et al. on Sep. 9, 1986; U.S. Pat. No. 4,673,402, entitled "Absorbent Articles With Dual-Layered Cores," which issued to Weisman et al. on Jun. 16, 1987; U.S. Pat. No. 4,834,735, entitled "High Density Absorbent Members Having Lower Density and Lower Basis Weight Acquisition Zones," which issued to Alemany et al. on May 30, 1989; U.S. Pat. No. 4,888,231, entitled "Absorbent Core Having A Dusting Layer," which issued to Angstadt on Dec. 19, 1989; U.S. Pat. No. 5,137,537, entitled "Absorbent Structure Containing Individualized, Polycarboxylic Acid Crosslinked Wood Pulp Cellulose Fibers," which issued to Herron et al. on Aug. 11, 1992; U.S. Pat. No. 5,147,345, entitled "High Efficiency Absorbent Articles For Incontinence Management," which issued to Young et al. on Sep. 15, 1992; U.S. Pat. No. 5,342,338, entitled "Disposable Absorbent Article For Low-Viscosity Fecal Material," issued to Roe on Aug. 30, 1994; U.S. Pat. No. 5,260,345, entitled "Absorbent Foam Materials For Aqueous Body Fluids and Absorbent Articles Containing Such Materials," which issued to DesMarais et al. on Nov. 9, 1993; U.S. Pat. No. 5,387,207, entitled "Thin-Until-Wet Absorbent Foam Materials For Aqueous Body Fluids And Process For Making Same," which issued to Dyer et al. on Feb. 7, 1995; and U.S. Pat. No. 5,650,222, entitled "Absorbent Foam Materials For Aqueous Fluids Made From high Internal Phase Emulsions Having Very High Water-To-Oil Ratios," which issued to DesMarais et al. on Jul. 22, 1997, all of which are hereby incorporated by reference herein.

The absorbent core may also have a multiple layered construction. A more detailed discussion of various types of multi-layered absorbent cores can be found in U.S. Pat. No. 5,669,894, entitled "Absorbent Members for Body Fluids having Good Wet Integrity and Relatively High Concentrations of Hydrogel-forming Absorbent Polymer," issued to Goldman et al. on Sept. 23, 1997; U.S. Pat. No. 6,441,266, entitled "Absorbent Members for Body Fluids using Hydrogel-forming Absorbent Polymer," issued to Dyer et al. on Aug. 26, 2002; U.S. Pat. No. 5,562,646, entitled "Absorbent Members for Body Fluids having Good Wet Integrity and Relatively High Concentrations of Hydrogel-forming Absorbent Polymer having High Porosity," issued to Goldman et al. on Oct. 10, 1996; European Pat. No. EP0565606B1, published on Mar. 8, 1995; U.S. Pat. Publication No. 2004/0162536A1 published Aug. 19, 2004; U.S. Pat. Publication No. 2004/0167486A1 published on Aug. 26, 2004; and PCT Publication No. WO 2006/015141 published on Feb. 9, 2006, which are all hereby incorporated by reference herein. In some embodiments, the absorbent article includes an absorbent core that is stretchable. In such a configuration, the absorbent core may be adapted to extend along with other materials of the chassis in longitudinal and/or lateral directions. The absorbent core can also be connected with the other components of the chassis various ways. For example, the diaper may include a "floating core" configuration or a "bucket" configuration wherein the diaper includes an anchoring system that can be configured to collect forces tending to move the article on the wearer. Such an anchoring system can also be configured to anchor itself to a body of a wearer by contacting various parts of the body. In this way, the anchoring system can balance the collected moving forces with holding forces obtained from the anchoring. By balancing the collected moving forces with the obtained holding forces, the anchoring system can at least assist in holding the disposable wearable absorbent article in place on a wearer. A more detailed discussion of various floating and/or bucket core configurations can be found in U.S. provisional patent application number 60/811,700, entitled "Absorbent Article Having a Multifunctional Containment Member," filed on June 7, 2006; U.S. Application No. 11/599,851; and U.S. Application No. 11/599,862, which are all hereby incorporated by reference herein.

The diapers according to the present disclosure can also include other features such as elastically extensible side panels. The side panels may be joined at seams to form the waist opening and the leg openings. The diapers may also includes leg elastics 216, such as shown in Fig. 8, and an elastic waist region to enhance the fits around the legs and waist of the wearer. Example leg elastic and leg cuff embodiments are disclosed in, for example, U.S. Patent No. 4,695,278 issued to Lawson on September 22, 1987; and U.S. Patent No. 4,795,454 issued to Dragoo on January 3, 1989.

In addition to the backsheet, topsheet, absorbent core, acquisition layer, and other diaper components, graphics may also be printed on substrates used as printed component material to construct the fastening elements on the diaper, such as for example, a landing zone. Depending on the particular configuration, it is to be appreciated that various types of fastening elements may be used with the diaper. In one example, the fastening elements include hook & loop fasteners, such as those available from 3M or Velcro Industries. In other examples, the fastening elements include adhesives and/or tap tabs, while others are configured as a macrofastener or hook (e.g., a MACRO or "button-like" fastener). Some exemplary fastening elements and systems are disclosed in U.S. Pat. No. 3,848,594, entitled "Tape Fastening System for Disposable Diaper," which issued to Buell on Nov. 19, 1974; U.S. Pat. No. B1 4,662,875, entitled "Absorbent Article," which issued to Hirotsu et al. on May 5, 1987; U.S. Pat. No. 4,846,815, entitled "Disposable Diaper Having An Improved Fastening Device," which issued to Scripps on Jul. 11, 1989; U.S. Pat. No. 4,894,060, entitled "Disposable Diaper With Improved Hook Fastener Portion," which issued to Nestegard on Jan. 16, 1990; U.S. Pat. No. 4,946,527, entitled "Pressure-Sensitive Adhesive Fastener And Method of Making Same," which issued to Battrell on Aug. 7, 1990; and U.S. Pat. No. 5,151,092, issued to Buell on Sep. 29, 1992; and U.S. Pat. No. 5,221,274, which issued to Buell on Jun. 22, 1993, which are all hereby incorporated by reference herein. Additional examples of fasteners and/or fastening elements are discussed in U.S. Pat. Nos. 6,482,191, 6,251,097 and 6,432,098; U.S. Patent Application Serial No. 11/240,943, entitled, "Anti-Pop Open Macrofasteners" filed on September 30, 2005; and U.S. Patent Application Serial No. 11/240,838, entitled, "A Fastening System Having Multiple Engagement Orientations", filed on September 30, 2005, which are all hereby incorporated by reference herein. Other fastening systems are described in more detail in U.S. Pat. No. 5,595,567 issued to King et al. on Jan. 21, 1997 and U.S. Pat. No. 5,624,427 issued to Bergman et al. on Apr. 29, 1997, both of which are entitled "Nonwoven Female Component For Refastenable Fastening Device." Yet other fastening systems are described in U.S. Pat. Nos. 5,735,840 and 5,928,212, both of which issued to Kline et al. and are entitled "Disposable Diaper With Integral Backsheet Landing Zone," which are both hereby incorporated by reference herein. The fastening system may also provide a means for holding the article in a disposal configuration as disclosed in U.S. Pat. No. 4,963,140, which issued to Robertson et al. on Oct. 16, 1990, which is hereby incorporated by reference herein.

The foregoing description of the diaper shown in Figs. 7 and 8, illustrate that a repeating series of graphics G1-Gn and identical graphics IG may be printed according to the methods and apparatuses disclosed herein on substrates, which may be referred to as component graphic material, to construct various components, such as for example, backsheets, topsheets, absorbent cores, acquisition layers, landing zones, and other fastening elements. In addition, the graphics may be printed on the body facing surface, the garment facing surface, or both surfaces of such components.

As previously mentioned, in some embodiments of the absorbent product, the variable graphics G1-Gn on the absorbent articles are different from each other in terms of graphic design, and the identical graphics IG on the absorbent articles are identical to each other in terms of graphic design. Herein, "different in terms of graphic design" means that graphics are intended to be different when viewed by users or consumers with normal attentions. And, "identical in terms of graphic design" means that graphics are intended to be the same when viewed by users or consumers with normal attentions. Thus, two graphics having a graphic difference(s) which are unintentionally caused due to a problem(s) or an error(s) in a manufacture process, for example, are not different from each other in terms of graphic design. The graphic design is determined by, for example, the color(s) used in the graphic (individual pure ink colors as well as built process colors), the sizes of the entire graphic (or components of the graphic), the positions of the graphic (or components of the graphic), the movements of the graphic (or components of the graphic), the geometrical shapes of the graphic (or components of the graphics), the number of colors in the graphic, the variations of the color combinations in the graphic, the number of graphics printed, the disappearance of color(s) in the graphic, and the contents of text messages in the graphic.

It should be appreciated that although a package may contain absorbent articles which have the graphics G1-Gn different from each other, the package may also contain, if desired, one or more additional absorbent article(s) which has a graphic that is the same as one the other graphics in the package. In other words, the absorbent product may include at least n absorbent articles, in a series, which have the graphics G1-Gn different from each other, and can include an additional absorbent article(s) each having the same graphic(s).

It should be appreciated that printed variable graphics and/or identical graphics may be other types that are permanent or active graphics. Active graphics are graphics that are configured to appear or disappear upon various types of triggering mechanisms or stimuli, such as for example, moisture (e.g. aquachromic ink graphics), temperature change (e.g. thermochromic ink graphics), and/or light (e.g. photochromic ink graphics, UV or IR light).

It is also to be appreciated that the position of the variable graphics G1-Gn and/or identical graphics IG may be registered within a predetermined area of the absorbent articles such that each of the variable graphics G1-Gn and/or identical graphics IG appear in an intended position (or the predetermined area) in each absorbent articles without unintentional variation. In the embodiment shown in Fig. 7, the variable graphics G1-Gn are registered in the first waist region of the absorbent article.

The identical graphics IG and/or variable graphics G1-Gn of the absorbent articles may also have a predetermined association. Herein, "association" refers to a relationship which can conceptually bond a plurality of graphics. The predetermined association may be formed by the graphic designs of the n graphics. The predetermined association may include a predetermined order and/or a common theme.

In some embodiments, the predetermined association includes a predetermined order, and the n absorbent articles are stacked in the package in accordance with the predetermined order. The predetermined order may include an order illustrating story, an order for daily activity, an order for educational training, an order for sequential indication, an order of usage instruction, an order illustrating child care tips, and an order of sales promotion. In some embodiments, each absorbent article carries one step or stage in a predetermined order in the graphic, and the predetermined order is completed by the n graphics of the n absorbent articles. In embodiments where the graphics illustrate a story, the story may include a children's story and a cartoon story such as Aesop's Fables, nursery rhymes, and the like. In some embodiments, an absorbent product may include absorbent articles in a single package wherein each absorbent article including graphics illustrating different stories or nursery rhymes. For example, an absorbent article may include a graphic G1 illustrating a first nursery rhyme, such as Jack and Jill, and an adjacent absorbent article in the package may include a graphic G2 illustrating a second nursery rhyme, such as the Cat and the Fiddle, and so on up to graphic Gn. In some embodiments, an absorbent product may include absorbent articles in a single package wherein each absorbent product may include portions of stories or nursery rhymes. For example and as discussed above with reference Fig. 3E, an absorbent article in the package may include a graphic G1 illustrating a first portion of a first nursery rhyme, such as "Hey, Diddle, Diddle!", and an adjacent absorbent article may include a graphic G2 illustrating a second portion of the first nursery rhyme, such as "The Cat and the Fiddle!" and a next adjacent absorbent article may include a graphic G3 illustrating a third portion of the first nursery rhyme, such as "The Cow Jumped over the Moon!", continuing until the nursery rhyme is completed. Additional absorbent articles in the package may have graphics that repeat the first nursery rhyme or may include graphics that illustrate portions of other nursery rhymes. The order for daily activity may include, for example, eating foods, wearing (or changing) clothes, taking a bath, a toilet activity, making an object, cooking a food, sleeping, and growing a plant. For example, when changing clothes, the absorbent article may have a graphic G1 which shows the first step of changing clothes (e.g., taking off a pair of pajamas), the absorbent article may have a graphic G2 which shows the second step (e.g., taking off a used underwear), the absorbent article may further have a graphic G3 which shows the third step (e.g., putting on a clean underwear), and the like. In addition, the order for daily activity may be shown together with preferred times for such activities in the graphics G1-Gn (e.g., 8:00 PM for sleeping). The order for educational training may include, for example, a puzzle or quiz on mathematics, characters (e.g., numbers and letters) which are decorated or undecorated, shapes of goods, combinations of colors, and a pattern recognition for intelligence development. The order for sequential indication may include, for example, a sequential symbol. The sequential symbol may indicate the number of the remaining absorbent articles in the package when the absorbent articles are consumed. Any sequential symbol including numbers (e.g., 1-60) and letters (e.g., A-Z) can be used. Such numbers and letters can also be used as an educational tool for kids. The order of usage instruction can include any information for users to effectively use absorbent articles. The order for child care tips can include any information for users (or care givers) to effectively take care of babies or children. The order for sales promotion can include any information for effectively advertising the absorbent articles to consumers.

In some embodiments, the predetermined association may include a common theme, and the n absorbent articles may be stacked in the package in a randomly selected order. The common theme can be any theme which is consistently expressed in the n graphics. The common theme may include cartoon characters (e.g., one cartoon character is doing different activities such as playing, eating, taking a bath, and the like, or a plurality of different cartoon characters are doing same/different activities), transportation means (e.g., cars, trains, ship, planes, etc.), animals (e.g., dogs, cats, rabbits, etc.), fruits (e.g., bananas, oranges, apples, etc.), vegetables (e.g., carrots, pumpkins, potatoes, etc.), plants (e.g., tulips, morning glories, roses, etc.), and seasonal themes (e.g., snowmen, etc.).

Although the printing apparatuses and methods disclosed herein may be an offline printing processes (i.e., the printing process is a not part of a diaper manufacture process), the printing apparatuses and methods disclosed herein are also applicable to an online processes. In the offline printing process, the printed substrate may exit the printing apparatus and be wound on a roll. The wound, printed substrate may then be used in a separate diaper manufacture process.

As discussed above, the printing apparatuses disclosed herein may be used in a process to manufacture absorbent products including pluralities of absorbent articles with one or more repeating series of variable graphics (G1-Gn) and identical graphics IG contained in packages, wherein the graphics G1-Gn are different from each other. In a first step of an example process, a substrate is fed in a machine direction MD onto the rotating central impression cylinder of the printing apparatus having a plurality of printing stations disposed about the outer surface of the central impression cylinder. Each variable graphic printing station may include n printing plates disposed on a print cylinder and are adapted to print a repeating series of n graphics (G1-Gn) in the MD direction on the substrate. And each constant graphic printing station may include one or printing plates disposed on a print cylinder and are adapted to print a repeating series of identical graphics adjacent the variable graphics. The printing stations may also be configured for halftone printing and configured to print different colors. In a second step, the substrate is moved past each printing station on the rotating central impression cylinder. In a third step, ink is transferred from the printing plates on the printing stations to substrate. In some embodiments, the ink is transferred from each printing station in rows of dots. In addition, the rows of dots from each printing station may be printed at different screen angles so the graphics appear in different colors. In some embodiments, four printing stations are configured to print cyan, magenta, yellow, and black colors at screen angles of 15°, 75°, 0° or 90°, 45°, respectively. In a fourth step, the printed substrate exits the printing apparatus and is slit and then rewound onto a finished roll. The finished roll may later be moved to a diaper manufacturing line and used as printed component material to construct printed components of an absorbent article, such as for example, a backsheet, topsheet, absorbent core, acquisition layer, and/or landing zone. In a fifth step, the individual printed components are modified or otherwise combined with other advancing substrates or webs and/or individual component parts. In a sixth step, the advancing webs are subjected to a final knife cut and separated into discrete absorbent articles, such as diapers, wherein a repeating series of n adjacent articles each have different graphics and identical graphics printed thereon. In a seventh step, one or more, or a portion of, the series of n absorbent articles are folded, stacked, and placed in a package.

While the above apparatuses and methods for printing graphics on a substrate are described in the context of substrates used to construct components of various types of absorbent articles, it is to be appreciated that apparatuses and methods according to the present disclosure can be utilized to print substrates used to construct other types of components. In one instance, the above apparatuses and methods can be configured to print graphics on substrates used to make components for packaging. For example, a substrate, such as plastic or paper, can be printed with one or more series of graphics as described above, wherein the plastic or paper substrate is then used to manufacture packages of consumer products. In a particular example, the substrate can be printed with a series of m different graphics and a series identical graphics, and the printed substrate, in turn, is used to construct a series of m packages (4001-400m) having first package graphics (G1-Gm) that are different from each other and second package graphics IG that are identical to each other, wherein m can be a number of 2 or greater, as shown in Fig. 9. It is to be appreciated that the packages 4001-400m may have various different types of configurations. Such packages (4001-400m) can be filled with consumer products and placed on pallets for shipping and/or display. In yet another example, a series of m packages (4001-400m) having graphics (G1-Gm) that are different from each other can have graphics that have a predetermined association with graphics printed on consumer products, such as absorbent articles, contained within the packages. For example, a series of absorbent products may include a first package in a series which may have graphics (e.g. cartoon character, story line, or nursery rhyme) printed thereon, and absorbent articles contained within the first package may include printed graphics having a predetermined association with the graphics printed on the first package. A second package in the series may have different graphics from those printed on the first package, and the absorbent articles contained within the second package may include graphics printed thereon having a predetermined association with the graphics printed on the second package. In still another example, one or more series of consumer products, such as any of the absorbent articles (3001-300n) described herein, such as those for example discussed above with reference to Figs. 6A and 6B, may be contained within the series of m packages (4001-400m), which may also include graphics (G1-Gm) that are different from each other.

As discussed above, substrates may be printed with graphics utilizing a halftone printing process. The following provides a test method for detecting and analyzing graphics printed in accordance with the processes and apparatuses disclosed herein with a halftone process.

### Test Method

Carefully remove the printed substrate from the article taking care not to deform the substrate's dimensions. Typically layers can be separated using a flash-freezing spray such as Cyto-Freeeze (Control Co. TX) or gently heating the article to release the adhesives. Lay the specimen flat on a lab bench with the printed side facing up, and draw a reference line centered along the longitudinal length of the specimen. Identify a one square inch test area that includes a printed image where either 1) a color is constructed with overlapping print, where dots of at least one screen color can be discerned or 2) halftone printing where dots of the screen color can be discerned. Draw a first auxiliary line, perpendicular to the reference line, which passes through the test area. Next, place the substrate, printed side down, on the scanning surface of a flat bed scanner (for example an Epson Perfection V500 Photo scanner), close the lid and scan the identified test region at least 4800 dpi and 24-bit color depth in reflectance mode.

Examine the digital image within a graphics program such as Image J (National Institute of Health, USA). Rotate the digital image as necessary to align the first auxiliary line horizontally. Visually identify a linear arrangement of printed screen dots of a specific first color, for example 140 in Figure 5. Using the software, draw a second auxiliary line through the center of the chosen screen dots which also intersects the first auxiliary line. To facilitate the angle measurement, the right direction of the first auxiliary line is taken to be 0° and the left direction 180°. The arc of the angle starts at 0° and arcs counter-clockwise to 180° (note all measured angles will be 180° or less). Once again, using the software, measure the angle between the first and second auxiliary lines to ± 1.0 degree.

Next, an angle for a second distinct printed screen color is measured in like fashion. The second angle can be measured within the same test area, or if needed, a second test area can be chosen, scanned, and measured, following the same procedure outlined above.

Compare the angles of the two measured printed screen colors, calculating the difference between them to ± 1.0 degree. Repeat the angle measurements, using corresponding test areas and colors for a least 3 articles. Report the average angle difference to ± 1.0 degree.

Table 4 below shows exemplary data gathered using the test method described above by measuring the screen angles of ink dots printed on absorbent articles:

**Table 4**

| Brand Product Size | Printed Layer | Color 1 | Color 2 | Replicates (Δ angle degrees) | | | Average Degrees |
|---|---|---|---|---|---|---|---|
| | | | | 1 | 2 | 3 | |
| White Cloud Training Pants 3T-4T | Film Backsheet | Magenta | Cyan | 28.24 | 28.92 | 29.05 | 28.7 |
| Huggies Supreme Natural Fit 3 | Nonwoven Cover | Green | Cyan | 31.40 | 30.38 | 30.58 | 30.8 |
| Pampers Cruisers 3 | Film Backsheet | Cyan | Yellow | 13.45 | 13.27 | 14.05 | 13.6 |

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

## Claims

1. An apparatus (100) for printing disposable absorbent articles comprising:
a central impression cylinder (102) defining an outer circumferential surface;
a constant graphic printing station (104') positioned adjacent the outer circumferential surface (106) of the central impression cylinder (102), the constant graphic printing station (104') comprising:
a print cylinder (112') defining an outer circumferential surface (120) defining a first circumferential length;
a constant graphic printing pattern (2000) disposed on the outer circumferential surface of the print cylinder;
an ink supply (124); and
an anilox roller (126) operably connected with the ink supply (124) and the print cylinder (112') wherein the anilox roller (126) is adapted to deposit ink from the ink supply onto the constant graphic printing pattern (2000);
a first variable graphic printing station (104"a) positioned adjacent the outer circumferential surface (106) of the central impression cylinder (102);
a second variable graphic printing station (104"b) positioned adjacent the outer circumferential surface (106) of the central impression cylinder (102);
a third variable graphic printing station (104"c) positioned adjacent the outer circumferential surface (106) of the central impression cylinder (102);
wherein each variable graphic printing station (104"a, 104"b, 104"c) comprises:
a print cylinder (112") defining an outer circumferential surface (120) defining a second circumferential length;
a plurality of n variable printing patterns (200n) disposed on the outer circumferential surface of the print cylinder (112"), wherein n is 2 or
greater and wherein the n variable printing patterns are different from each other;
an ink supply (124); and
an anilox roller (126) operably connected with the ink supply (124) and the print cylinder (112") wherein the anilox roller is adapted to deposit ink from the ink supply onto the plurality of n variable printing patterns; and
wherein the constant graphic printing pattern (2000) is different from the n variable printing patterns (200n); and
wherein the second circumferential length is at least two times the first circumferential length.

2. The apparatus of claim 1, further comprising a fourth variable graphic printing station (104"d).

3. The apparatus of claim 2, wherein the first variable graphic printing station (104"a) is adapted to print cyan, the second variable graphic printing station (104"b) is adapted to print magenta, the third variable graphic printing station (104"c) is adapted to print yellow, and a fourth variable graphic printing station (104"d) is adapted to print black.

4. The apparatus of claim 3, wherein the first variable graphic printing station (104"a) is adapted to print a first ink color at a first screen angle, the second variable graphic printing station (104"b) is adapted to print a second ink color at a second screen angle, the third variable graphic printing station (104"c) is adapted to print a third ink color at a third screen angle, and the fourth variable graphic printing station (104"d) is adapted to print a fourth ink color at a fourth screen angle.

5. The apparatus of claim 1, the plurality of variable graphic printing stations further comprising a plurality of n printing plates (100n) disposed on the print cylinder (112"), and wherein the variable printing patterns (200n) are disposed on the plurality of n printing plates (100n).

6. The apparatus according to any of the preceding claims, further comprising a second constant graphic printing station (104'b) positioned adjacent the outer circumferential surface (106) of the central impression cylinder (102), the second constant graphic printing station (104'b) comprising:
a print cylinder (112') defining an outer circumferential surface (120);
a second constant graphic printing pattern (2000) disposed on the outer circumferential surface of the print cylinder;
an ink supply (124); and
an anilox roller (126) operably connected with the ink supply (124) and the print cylinder (112') wherein the anilox roller (126) is adapted to deposit ink from the ink supply onto the second constant graphic printing pattern (2000); and
wherein the second constant graphic printing pattern (2000) is different from the first constant graphic printing patterns (200n).

7. The apparatus of claim 6, further comprising a third constant graphic printing station (104'c) positioned adjacent the outer circumferential surface (106) of the central impression cylinder (102), the third constant graphic printing station (104'c) comprising:
a print cylinder (112') defining on outer circumferential surface (120);
a plurality of identical printing patterns (2000) operably disposed on the outer circumferential surface of the print cylinder;
an ink supply (124); and
an anilox roller operably connected with the ink supply and the print cylinder wherein the anilox roller is adapted to deposit ink from the ink supply onto the plurality of identical printing patterns (2000); and
wherein the plurality of identical printing patterns of each constant graphic printing station (104', 104'b, 104'c) are the same; and
wherein the constant graphic printing stations (104', 104'b, 104'c) are adapted to print ink colors different from each other.

8. A method for producing disposable absorbent articles (158) comprising the steps of:
feeding a substrate (108) onto a rotating central impression cylinder (102);
moving the substrate (108) past a constant graphic printing station (104') arranged adjacent an outer surface (106) of the central impression cylinder, wherein the constant graphic printing station (104') includes a print cylinder (112') with a plurality of identical printing patterns (2000) operably disposed thereon;
rotating the print cylinder (112') of the constant graphic printing station (104') to print a series of identical graphics on the substrate;
moving the substrate (108) past a plurality of variable graphic printing stations (104") arranged around an outer surface (106) of the central impression cylinder (102), wherein each printing station includes a print cylinder (112") with n variable printing patterns (200n) operably disposed thereon, wherein n is 2 or greater and wherein the n variable printing patterns are different from each other and are different from the identical printing patterns;
rotating the print cylinders (112") of the variable graphic printing stations (104") to print a series of n graphics adjacent the identical graphics;
converting the substrate (108) into printed components of disposable absorbent articles (158); and
placing the disposable absorbent articles (158) into a package (156).

## Patentansprüche

1. Apparat (100) zum Bedrucken von Einweg-Absorptionsartikeln, umfassend:
einen zentralen Gegendruckzylinder (102), der eine äußere Umfangsoberfläche bestimmt;
eine konstante Grafikdruckstation (104'), die angrenzend an die äußere Umfangsoberfläche (106) des zentralen Gegendruckzylinders (102) angeordnet ist, wobei die konstante Grafikdruckstation (104') Folgendes umfasst:
einen Druckzylinder (112'), der eine äußere Umfangsoberfläche (120) bestimmt, die eine erste Umfangslänge bestimmt;
eine konstante Grafikdruckvorlage (2000), die auf der äußeren Umfangsoberfläche des Druckzylinders angeordnet ist;
eine Tintenzufuhr (124); und
eine Anilox-Walze (126), die operabel mit der Tintenzufuhr (124) und dem Druckzylinder (112') verbunden ist, wobei die Anilox-Walze (126) so konzipiert ist, dass sie Tinte aus der Tintenzufuhr auf die konstante Grafikdruckvorlage (2000) aufträgt;
eine erste variable Grafikdruckstation (104"a), die angrenzend an die äußere Umfangsoberfläche (106) des zentralen Gegendruckzylinders (102) angeordnet ist;
eine zweite variable Grafikdruckstation (104"b), die angrenzend an die äußere Umfangsoberfläche (106) des zentralen Gegendruckzylinders (102) angeordnet ist;
eine dritte variable Grafikdruckstation (104"c), die angrenzend an die äußere Umfangsoberfläche (106) des zentralen Gegendruckzylinders (102) angeordnet ist;
wobei jede variable Grafikdruckstation (104"a, 104"b, 104"c) Folgendes umfasst:
einen Druckzylinder (112"), der eine äußere Umfangsoberfläche (120) bestimmt, die eine zweite Umfangslänge bestimmt;
eine Mehrzahl von n variablen Druckvorlagen (200n), die an der äußeren Umfangsoberfläche des Druckzylinders (112") angeordnet sind, wobei n 2 oder größer ist und wobei sich die n variablen Druckvorlagen voneinander unterscheiden;
eine Tintenzufuhr (124); und
eine Anilox-Walze (126), die operabel mit der Tintenzufuhr (124) und dem Druckzylinder (112") verbunden ist, wobei die Anilox-Walze so konzipiert ist, dass sie Tinte aus der Tintenzufuhr auf die Mehrzahl von n variablen Druckvorlagen aufträgt; und
wobei sich die konstante Grafikdruckvorlage (2000) von den n variablen Druckvorlagen (200n) unterscheidet; und
wobei die zweite Umfangslänge mindestens zweimal so groß ist wie die erste Umfangslänge.

2. Apparat nach Anspruch 1, ferner umfassend eine vierte variable Grafikdruckstation (104"d).

3. Apparat nach Anspruch 2, wobei die erste variable Grafikdruckstation (104"a) zum Drucken von Cyan konzipiert ist, die zweite variable Grafikdruckstation (104"b) zum Drucken von Magenta konzipiert ist, die dritte variable Grafikdruckstation (104"c) zum Drucken von Gelb konzipiert ist und eine vierte variable Grafikdruckstation (104"d) zum Drucken von Schwarz konzipiert ist.

4. Apparat nach Anspruch 3, wobei die erste variable Grafikdruckstation (104"a) zum Drucken einer ersten Tintenfarbe unter einem ersten Rasterwinkel konzipiert ist, die zweite variable Grafikdruckstation (104"b) zum Drucken einer zweiten Tintenfarbe unter einem zweiten Rasterwinkel konzipiert ist, die dritte variable Grafikdruckstation (104"c) zum Drucken einer dritten Tintenfarbe unter einem dritten Rasterwinkel konzipiert ist und die vierte variable Grafikdruckstation (104"d) zum Drucken einer vierten Tintenfarbe unter einem vierten Rasterwinkel konzipiert ist.

5. Apparat nach Anspruch 1, wobei die Mehrzahl von variablen Grafikdruckstationen ferner eine Mehrzahl von n Druckplatten (100n) umfasst, die auf dem Druckzylinder (112") angeordnet sind, und wobei die variablen Druckvorlagen (200n) auf der Mehrzahl von n Druckplatten (100n) angeordnet sind.

6. Apparat nach einem der vorstehenden Ansprüche, ferner umfassend eine zweite konstante Grafikdruckstation (104'b), die angrenzend an die äußere Umfangsoberfläche (106) des zentralen Gegendruckzylinders (102) angeordnet ist, wobei die zweite konstante Grafikdruckstation (104'b) Folgendes umfasst:
einen Druckzylinder (112'), der eine äußere Umfangsoberfläche (120) bestimmt;
eine zweite konstante Grafikdruckvorlage (2000), die auf der äußeren Umfangsoberfläche des Druckzylinders angeordnet ist;
eine Tintenzufuhr (124); und
eine Anilox-Walze (126), die operabel mit der Tintenzufuhr (124) und dem Druckzylinder (112') verbunden ist, wobei die Anilox-Walze (126) so konzipiert ist, dass sie Tinte aus der Tintenzufuhr auf die zweite konstante Grafikdruckvorlage (2000) aufträgt; und
wobei sich die zweite konstante Grafikdruckvorlage (2000) von den ersten konstanten Grafikdruckvorlagen (200n) unterscheidet.

7. Apparat nach Anspruch 6, ferner umfassend eine dritte konstante Grafikdruckstation (104'c), die angrenzend an die äußere Umfangsoberfläche (106) des zentralen Gegendruckzylinders (102) angeordnet ist, wobei die dritte konstante Grafikdruckstation (104'c) Folgendes umfasst:
einen Druckzylinder (112'), der eine äußere Umfangsoberfläche (120) bestimmt;
eine Mehrzahl von identischen Druckvorlagen (2000), die operabel auf der äußeren Umfangsoberfläche des Druckzylinders angeordnet sind;
eine Tintenzufuhr (124); und
eine Anilox-Walze, die operabel mit der Tintenzufuhr und dem Druckzylinder verbunden ist, wobei die Anilox-Walze so konzipiert ist, dass sie Tinte aus der Tintenzufuhr auf die Mehrzahl von identischen Druckvorlagen (2000) aufträgt; und
wobei die Mehrzahl von identischen Druckvorlagen jeder konstanten Grafikdruckstation (104', 104'b, 104'c) identisch sind; und
wobei die konstanten Grafikdruckstationen (104', 104'b, 104'c) so konzipiert sind, dass sie Tintenfarben drucken, die sich voneinander unterscheiden.

8. Verfahren zur Herstellung von Einweg-Absorptionsartikeln (158), umfassend die folgenden Schritte:
Zuführen eines Substrats (108) auf einen rotierenden zentralen Gegendruckzylinder (102);
Vorbeiführen des Substrats (108) an einer konstanten Grafikdruckstation (104'), die angrenzend an eine Außenoberfläche (106) des zentralen Gegendruckzylinders angeordnet ist, wobei die konstante Grafikdruckstation (104') einen Druckzylinder (112') mit einer Mehrzahl von identischen Druckvorlagen (2000) aufweist, die operabel darauf angeordnet sind;
Drehen des Druckzylinders (112') der konstanten Grafikdruckstation (104'), um eine Folge von identischen Grafiken auf dem Substrat zu drucken;
Vorbeiführen des Substrats (108) an einer Mehrzahl von variablen Grafikdruckstationen (104"), die um eine Außenoberfläche (106) des zentralen Gegendruckzylinders (102) herum angeordnet sind, wobei jede Druckstation einen Druckzylinder (112") mit n variablen Druckvorlagen (200n) aufweist, die operabel darauf angeordnet sind, wobei n 2 oder größer ist und wobei sich die n variablen Druckvorlagen voneinander unterscheiden und von den identischen Druckvorlagen unterscheiden;
Drehen des Druckzylinders (112") der variablen Grafikdruckstationen (104"), um eine Folge von n Grafiken angrenzend an die identischen Grafiken zu drucken;
Umwandeln des Substrats (108) in bedruckte Bestandteile von Einweg-Absorptionsartikeln (158); und
Platzieren der Einweg-Absorptionsartikel (158) in eine Verpackung (156).

## Revendications

1. Appareil (100) pour imprimer des articles absorbants jetables comprenant :
un cylindre d'impression central (102) définissant une surface circonférentielle externe;
une station d'impression graphique constante (104') positionnée adjacente à la surface circonférentielle externe (106) du cylindre d'impression central (102), la station d'impression graphique constante (104) comprenant :
un cylindre d'impression (112') définissant une surface circonférentielle externe (120) définissant une première longueur circonférentielle ;
un motif d'impression graphique constante (2000) disposé sur la surface circonférentielle externe du cylindre d'impression ;
une alimentation d'encre (124) ; et
un rouleau anilox (126) attaché de manière fonctionnelle à l'alimentation d'encre (124) et au cylindre d'impression (112'), dans lequel le rouleau anilox (126) est adapté pour déposer de l'encre de l'alimentation d'encre sur le motif d'impression graphique constante (2000) ;
une première station d'impression graphique variable (104"a) positionnée adjacente à la surface circonférentielle externe (106) du cylindre d'impression central (102) ;
une deuxième station d'impression graphique variable (104"b) positionnée adjacente à la surface circonférentielle externe (106) du cylindre d'impression central (102) ;
une troisième station d'impression graphique variable (104"c) positionnée adjacente à la surface circonférentielle externe (106) du cylindre d'impression central (102) ;
dans lequel chaque station d'impression graphique variable (104"a, 104"b, 104"c) comprend :
un cylindre d'impression (112') définissant une surface circonférentielle externe (120) définissant une deuxième longueur circonférentielle ;
une pluralité de n motifs d'impression variables (200n) disposés sur la surface circonférentielle externe du cylindre d'impression (112"), dans lequel n est 2 ou plus et dans lequel les n motifs d'impression variables sont différents les uns des autres ;
une alimentation d'encre (124) ; et
un rouleau anilox (126) attaché de manière fonctionnelle à l'alimentation d'encre (124) et au cylindre d'impression (112"), dans lequel le rouleau anilox est adapté pour déposer de l'encre de l'alimentation d'encre sur la pluralité de n motifs d'impression variables ; et
dans lequel le motif d'impression graphique constante (2000) est différent des n motifs d'impression variables (200n) ; et
dans lequel la deuxième longueur circonférentielle est au moins deux fois la première longueur circonférentielle.

2. Appareil selon la revendication 1, comprenant en outre une quatrième station d'impression graphique variable (104"d).

3. Appareil selon la revendication 2, dans lequel la première station d'impression graphique variable (104"a) est adaptée pour imprimer du cyan, la deuxième station d'impression graphique variable (104"b) est adaptée pour imprimer du magenta, la troisième station d'impression graphique variable (104"C) est adaptée pour imprimer du jaune, et une quatrième station d'impression graphique variable (104"d) est adaptée pour imprimer du noir.

4. Appareil selon la revendication 3, dans lequel la première station d'impression graphique variable (104"a) est adaptée pour imprimer une première couleur d'encre selon un premier angle de trame, une deuxième station d'impression graphique variable (104"b) est adaptée pour imprimer une deuxième couleur d'encre selon un deuxième angle de trame, la troisième station d'impression graphique variable (104"c) est adaptée pour imprimer une troisième couleur d'encre selon un troisième angle de trame, et la quatrième station d'impression graphique variable (104"d) est adaptée pour imprimer une quatrième couleur d'encre selon un quatrième angle de trame.

5. Appareil selon la revendication 1, dans lequel la pluralité de stations d'impression graphique variable comprend en outre une pluralité de n plaques d'impression (100n) disposées sur le cylindre d'impression (112"), et dans lequel les motifs d'impression variables (200n) sont disposés sur la pluralité de n plaques d'impression (100n).

6. Appareil selon l'une quelconque des revendications précédentes, comprenant en outre une deuxième station d'impression graphique constante (104'b) positionnée adjacente à la surface circonférentielle externe (106) du cylindre d'impression central (102), la deuxième station d'impression graphique constante (104'b) comprenant :
un cylindre d'impression (112') définissant une surface circonférentielle externe (120) ;
un deuxième motif d'impression graphique constante (2000) disposé sur la surface circonférentielle externe du cylindre d'impression ;
une alimentation d'encre (124) ; et
un rouleau anilox (126) attaché de manière fonctionnelle à l'alimentation d'encre (124) et au cylindre d'impression (112'), dans lequel le rouleau anilox (126) est adapté pour déposer de l'encre de l'alimentation d'encre sur le deuxième motif d'impression graphique constante (2000) ; et
dans lequel le deuxième motif d'impression graphique constante (2000) est différent du premier motif d'impression graphique constante (200n).

7. Appareil selon la revendication 6, comprenant en outre une troisième station d'impression graphique constante (104'c) positionnée adjacente à la surface circonférentielle externe (106) du cylindre d'impression central (102), la troisième station d'impression graphique constante (104'c) comprenant :
un cylindre d'impression (112') définissant une surface circonférentielle externe (120) ;
une pluralité de motifs d'impression identiques (2000) disposés de manière fonctionnelle sur la surface circonférentielle externe du cylindre d'impression ;
une alimentation d'encre (124) ; et
un rouleau anilox attaché de manière fonctionnelle à l'alimentation d'encre et au cylindre d'impression, dans lequel le rouleau anilox est adapté pour déposer de l'encre de l'alimentation d'encre sur la pluralité de motifs d'impression identiques (2000) ; et
dans lequel la pluralité de motifs d'impression identiques de chaque station d'impression graphique constante (104', 104'b, 104'c) sont les mêmes ; et
dans lequel les stations d'impression graphique constante (104', 104'b, 104'c) sont adaptées pour imprimer des couleurs d'encre différentes les unes des autres.

8. Procédé de production d'articles absorbants jetables (158) comprenant les étapes consistant à :
alimenter un substrat (108) sur un cylindre d'impression central (102) en rotation;
déplacer le substrat (108) au-delà d'une station d'impression graphique constante (104') arrangée adjacente à une surface externe (106) du cylindre d'impression central, dans lequel la station d'impression graphique constante (104') inclut un cylindre d'impression (112') avec une pluralité de motifs d'impression identiques (2000) disposés de manière fonctionnelle dessus ;
faire tourner le cylindre d'impression (112') de la station d'impression graphique constante (104') pour imprimer une série de motifs identiques sur le substrat ;
déplacer le substrat (108) au-delà d'une pluralité de stations d'impression graphique variable (104") arrangées autour d'une surface externe (106) du cylindre d'impression central (102), dans lequel chaque station d'impression inclut un cylindre d'impression (112") avec n motifs d'impression variables (200n) disposés de manière fonctionnelle dessus, dans lequel n est égal ou supérieur à 2 et dans lequel les n motifs d'impression variables sont différents les uns des autres et sont différents des motifs d'impression identiques ;
faire tourner les cylindres d'impression (112") des stations d'impression graphique variable (104") pour imprimer une série de n motifs adjacents aux motifs identiques ;
convertir le substrat (108) en composants imprimés d'articles absorbants jetables (158) ; et
placer les articles absorbants jetables (158) dans un conditionnement (156).
